(19) Europäisches Patentamt
European Patent Office
Office européen des brevets

(11) EP 2 020 414 A1

(12) EUROPEAN PATENT APPLICATION

(43) Date of publication:
**04.02.2009 Bulletin 2009/06**

(51) Int Cl.:
***C07D 495/20*** *(2006.01)* ***A61K 31/438*** *(2006.01)*
***A61P 25/18*** *(2006.01)*

(21) Application number: **07384027.4**

(22) Date of filing: **20.06.2007**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(71) Applicant: **Laboratorios del Dr. Esteve S.A.**
**08041 Barcelona (ES)**

(72) Inventors:
- **Oberdorf, Christoph**
  **48161 Münster (DE)**
- **Schepmann, Dirk, Dr.**
  **48147 Münster (DE)**
- **Wünsch, Bernhard, Prof. Dr.**
  **48161 Münster (DE)**
- **Zamanillo-Castanedo, Daniel, Dr.**
  **08041 Barcelona (ES)**

(74) Representative: **Peters, Hajo et al**
**Graf von Stosch Patentanwaltsgesellschaft mbH**
**Prinzregentenstrasse 22**
**80538 München (DE)**

(54) **spiro[piperidine-4,4'-thieno[3,2-c]pyran] derivatives and related compounds as inhibitors of the sigma receptor for the treatment of psychosis**

(57) The present invention relates to compounds having pharmacological activity towards the sigma (σ) receptor, and more particularly to some thieno-pyrano-pyrazole derivatives, to processes of preparation of such compounds, to pharmaceutical compositions comprising them, and to their use in therapy and prophylaxis, in particular for the treatment of psychosis or pain.

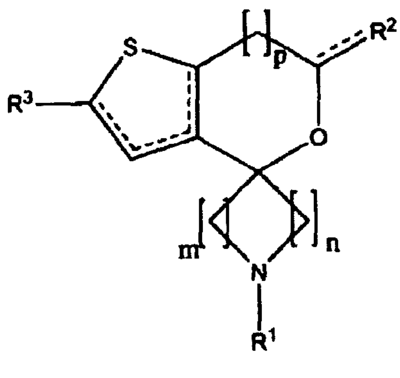

or

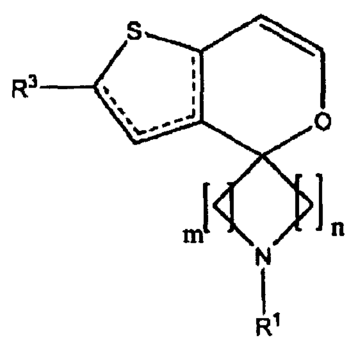

(I) (Ia)

EP 2 020 414 A1

**Description**

## FIELD OF THE INVENTION

The present invention relates to compounds having pharmacological activity towards the sigma (σ) receptor, and more particularly to some thieno-pyrano-pyrazole derivatives, to processes of preparation of such compounds, to pharmaceutical compositions comprising them, and to their use in therapy and prophylaxis, in particular for the treatment of psychosis.

## BACKGROUND OF THE INVENTION

The search for new therapeutic agents has been greatly aided in recent years by better understanding of the structure of proteins and other biomolecules associated with target diseases. One important class of these proteins is the sigma (σ) receptor, a cell surface receptor of the central nervous system (CNS) which may be related to the dysphoric, hallucinogenic and cardiac stimulant effects of opioids. From studies of the biology and function of sigma receptors, evidence has been presented that sigma receptor ligands may be useful in the treatment of psychosis and movement disorders such as dystonia and tardive dyskinesia, and motor disturbances associated with Huntington's chorea or Tourette's syndrome and in Parkinson's disease (Walker, J.M. et al, Pharmacological Reviews, 1990, 42, 355). It has been reported that the known sigma receptor ligand rimcazole clinically shows effects in the treatment of psychosis (Snyder, S.H., Largent, B.L. J. Neuropsychiatry 1989, 1, 7). The sigma binding sites have preferential affinity for the dextrorotatory isomers of certain opiate benzomorphans, such as (+)SKF 10047, (+)cyclazocine, and (+)pentazocine and also for some narcoleptics such as haloperidol.

The sigma receptor has at least two subtypes, which may be discriminated by stereoselective isomers of these pharmacoactive drugs. SKF 10047 has nanomolar affinity for the sigma 1 (σ-1) site, and has micromolar affinity for the sigma (σ-2) site. Haloperidol has similar affinities for both subtypes. Endogenous sigma ligands are not known, although progesterone has been suggested to be one of them. Possible sigma-site-mediated drug effects include modulation of glutamate receptor function, neurotransmitter response, neuroprotection, behavior, and cognition (Quirion, R. et al. Trends Pharmacol. Sci., 1992, 13:85-86). Most studies have implied that sigma binding sites (receptors) are plasmalemmal elements of the signal transduction cascade. Drugs reported to be selective sigma ligands have been evaluated as antipsychotics (Hanner, M. et al. Proc. Natl. Acad. Sci., 1996, 93:8072-8077). The existence of sigma receptors in the CNS, immune and endocrine systems have suggested a likelihood that it may serve as link between the three systems.

There is still a need to find compounds that have pharmacological activity towards the sigma receptor, being both effective and selective, and having good "drugability" properties, i.e. good pharmaceutical properties related to administration, distribution, metabolism and excretion.

The closest technique known comprises benzimidazoles of WO2003035065 for the inhibition of kinases. Tetrahydro-pyranopyrazole compounds displaying cannabinoid modulating activity are disclosed in WO2007001939 and FR2875230. None of the them presents spiro-pyrano-pyrazole variants or analogues.

Spiropiperidines are known as potent ligands to sigma receptors (Maier et al, J Med Chem, 2002, 45, 438-448 and Maier et al, J Med Chem, 2002, 45, 4923-4930). However, such spiropiperidines show benzofuran and benzopyran rings.

## SUMMARY OF THE INVENTION

We have now found a family of structurally distinct thieno-pyrano-pyrazole derivatives which are particularly selective inhibitors of the sigma receptor.

The invention is directed to compounds of general formula (I) or (Ia),

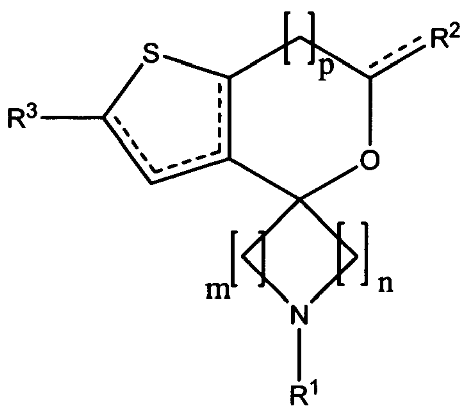

or

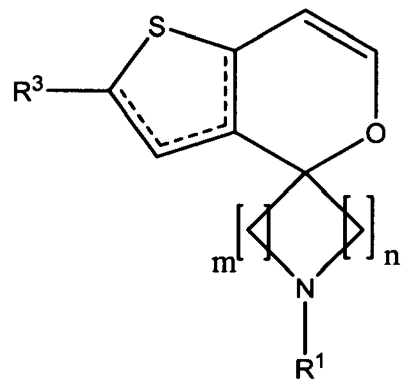

(I) (Ia)

wherein

m is selected from 1, 2 or 3, n is selected from 1, 2 or 3, and m + n is either 3, 4 or 5;

p is selected from 0 or 1;

the dotted lines ......... are either a double or a single bond;

$R^1$ is selected from hydrogen; an optionally at least monosubstituted, linear or branched $C_{1-18}$-aliphatic group; an optionally at least monosubstituted aryl; an optionally at least monosubstituted heterocyclyl; an optionally at least monosubstituted cycloalkyl; an optionally at least monosubstituted alkyl-aryl; an optionally at least monosubstituted alkyl-heterocyclyl; or an optionally at least monosubstituted alkyl-cycloalkyl;

$R^2$ is selected from hydrogen; an optionally at least monosubstituted, linear or branched $C_{1-6}$-aliphatic group; $(CH_2)_qCN$ with q being 0, 1, 2, 3 or 4; O-R with R being H or an optionally at least monosubstituted, linear or branched $C_{1-6}$-aliphatic group; =O; $(CH_2)_sCOR^*$; $(CH_2)_sNR^*R^{**}$; $CONR^*R^{**}$; $(CH_2)_sCO_2R^*$; $CH{=}NOR^*$; $(CH_2)_sOR^*$; with

R* being H; or an optionally at least monosubstituted, linear or branched $C_{1-6}$-aliphatic group;
R** being an optionally at least monosubstituted, linear or branched $C_{1-6}$-aliphatic group;
s being 0, 1, or 2;

$R^3$ is selected from hydrogen; an optionally at least monosubstituted, linear or branched $C_{1-18}$-aliphatic group; $NO_2$; $SO_3H$; COR'''; $(CH_2)_rNR'R''$; $CO_2R'$; an optionally at least monosubstituted alkyl-aryl with alkyl optionally being substituted by OH; an optionally at least monosubstituted alkyl-heterocyclyl with alkyl optionally being substituted by OH; or an optionally at least monosubstituted alkyl-cycloalkyl with alkyl optionally being substituted by OH; with

R' being H; or an optionally at least monosubstituted, linear or branched $C_{1-6}$-aliphatic group;
R'' being an optionally at least monosubstituted, linear or branched $C_{1-6}$-aliphatic group;
R''' being H; an optionally at least monosubstituted, linear or branched $C_{1-6}$-aliphatic group; or an optionally at least monosubstituted aryl, an optionally at least monosubstituted heterocyclyl, or an optionally at least mono-substituted cycloalkyl;
r being 0, 1, or 2;

optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In the context of this invention, alkyl radical or group is understood as meaning saturated and unsaturated,

linear or branched hydrocarbons, which can be unsubstituted or mono- or polysubstituted. Thus unsaturated alkyl is understood to encompass alkenyl and alkinyl groups, like e.g. $-CH=CH-CH_3$ or $-C\equiv C-CH_3$, while saturated alkyl encompasses e.g. $-CH_3$ and $-CH_2-CH_3$. In these radicals, $C_{1-2}$-alkyl represents C1- or C2-alkyl, $C_{1-3}$-alkyl represents C1-, C2- or C3-alkyl, $C_{1-4}$-alkyl represents C1-, C2-, C3- or C4-alkyl, $C_{1-5}$-alkyl represents C1-, C2-, C3-, C4-, or C5-alkyl, $C_{1-6}$-alkyl represents C1-, C2-, C3-, C4-, C5- or C6-alkyl, $C_{1-7}$-alkyl represents C1-, C2-, C3-, C4-, C5-, C6- or C7-alkyl, $C_{1-8}$-alkyl represents C1-, C2-, C3-, C4-, C5-, C6-, C7- or C8-alkyl, $C_{1-10}$-alkyl represents C1-, C2-, C3-, C4-, C5-, C6-, C7-, C8-, C9-or C10-alkyl and $C_{1-18}$-alkyl represents C1-, C2-, C3-, C4-, C5-, C6-, C7-, C8-, C9-, C10-, C11-, C12-, C13-, C14-, C15-, C16-, C17- or C18-alkyl. The alkyl radicals are preferably methyl, ethyl, vinyl (ethenyl), propyl, allyl (2-propenyl), 1-propinyl, methylethyl, butyl, 1-methylpropyl, 2-methylpropyl, 1,1-dimethylethyl, pentyl, 1,1-dimethylpropyl, 1,2-dimethylpropyl, 2,2-dimethylpropyl, hexyl, 1-methylpentyl, if substituted also $CHF_2$, $CF_3$ or $CH_2OH$ etc.

In the context of this invention aliphatic group or radical includes alkyl (saturated), alkenyl (unsaturated alkyl) and alkinyl (unsaturated alkyl) and thus is synonymous for: saturated or unsaturated alkyl (see above).

In the context of this invention cycloalkyl radical or group is understood as meaning saturated and unsaturated (but not aromatic) cyclic hydrocarbons (without a heteroatom in the ring), which can be unsubstituted or mono- or polysubstituted. Furthermore, $C_{3-4}$-cycloalkyl represents C3- or C4-cycloalkyl, $C_{3-5}$-cycloalkyl represents C3-, C4- or C5-cycloalkyl, $C_{3-6}$-cycloalkyl represents C3-, C4-, C5- or C6-cycloalkyl, $C_{3-7}$-cycloalkyl represents C3-, C4-, C5-, C6- or C7-cycloalkyl, $C_{3-8}$-cycloalkyl represents C3-, C4-, C5-, C6-, C7- or C8-cycloalkyl, $C_{4-5}$-cycloalkyl represents C4- or C5-cycloalkyl, $C_{4-6}$-cycloalkyl represents C4-, C5- or C6-cycloalkyl, $C_{4-7}$-cycloalkyl represents C4-, C5-, C6- or C7-cycloalkyl, $C_{5-6}$-cycloalkyl represents C5- or C6-cycloalkyl and $C_{5-7}$-cycloalkyl represents C5-, C6- or C7-cycloalkyl. However, mono- or polyunsaturated, preferably monounsaturated, cycloalkyls also in particular fall under the term cycloalkyl as long as the cycloalkyl is not an aromatic system. The alkyl and cycloalkyl radicals are preferably methyl, ethyl, vinyl (ethenyl), propyl, allyl (2-propenyl), 1-propinyl, methylethyl, butyl, 1-methylpropyl, 2-methylpropyl, 1,1-dimethylethyl, pentyl, 1,1-dimethylpropyl, 1,2-dimethylpropyl, 2,2-dimethylpropyl, hexyl, 1-methylpentyl, cyclopropyl, 2-methylcyclopropyl, cyclopropylmethyl, cyclobutyl, cyclopentyl, cyclopentylmethyl, cyclohexyl, cycloheptyl, cyclooctyl, and also adamantly.

In the context of this invention alkyl-cycloalkyl is understood as meaning a cycloalkyl group (see above) being connected to another atom through a $C_{1-6}$-alkyl group (see above), whereas the $C_{1-6}$-alkyl-group is always saturated and unsubstituted, and linear or branched.

In connection with alkyl or aliphatic group - unless defined otherwise - the term substituted in the context of this invention is understood as meaning replacement of at least one hydrogen radical by F, Cl, Br, I, $NH_2$, SH or OH, "polysubstituted" radicals being understood as meaning that the replacement takes effect both on different and on the same atoms several times with the same or different substituents, for example three times on the same C atom, as in the case of $CF_3$, or at different places, as in the case of e.g. $-CH(OH)-CH=CH-CHCl_2$.

The term $(CH_2)_{3-6}$ is to be understood as meaning $-CH_2-CH_2-CH_2-$, $-CH_2-CH_2-CH_2-CH_2-$, $-CH_2-CH_2-CH_2-CH_2-CH_2-$ and $-CH_2-CH_2-CH_2-CH_2-CH_2-CH_2-$, $(CH_2)_{1-4}$ is to be understood as meaning $-CH_2-$, $-CH_2-CH_2-$, $-CH_2-CH_2-CH_2-$ and $-CH_2-CH_2-CH_2-CH_2-$, $(CH_2)_{4-5}$ is to be understood as meaning $-CH_2-CH_2-CH_2-CH_2-$ and $-CH_2-CH_2-CH_2-CH_2-CH_2-$, etc.

An aryl radical or group is understood as meaning ring systems with at least one aromatic ring but without heteroatoms even in only one of the rings. Examples are phenyl, naphthyl, fluoranthenyl, fluorenyl, tetralinyl or indanyl, in particular 9H-fluorenyl or anthracenyl radicals, which can be unsubstituted or monosubstituted or polysubstituted.

In the context of this invention alkyl-aryl is understood as meaning an aryl group (see above) being connected to another atom through a $C_{1-6}$-alkyl-group (see above), whereas the $C_{1-6}$-alkyl-group is always saturated and unsubstituted, and linear or branched.

A heterocyclyl radical or group is understood as meaning heterocyclic ring systems, saturated or unsaturated ring which contains one or more heteroatoms from the group consisting of nitrogen, oxygen and/or sulfur in the ring and can also be mono- or polysubstituted. Examples which may be mentioned from the group of heteroaryls are furan, benzofuran, thiophene, benzothiophene, pyrrole, pyridine, pyrimidine, pyrazine, quinoline, isoquinoline, phthalazine, benzo-1,2,5-thiadiazole, benzothiazole, indole, benzotriazole, benzodioxolane, benzodioxane, carbazole and quinazoline.

In the context of this invention alkyl-heterocylyl is understood as meaning a heterocyclyl group (see above) being connected to another atom through a $C_{1-6}$-alkyl group (see above), whereas the $C_{1-6}$-alkyl-group is always saturated and unsubstituted, and linear or branched.

In connection with aryl or alkyl-aryl, cycloalkyl or alkyl-cycloalkyl, heterocyclyl or alkyl-heterocyclyl, substituted is understood - unless defined otherwise - as meaning substitution of the ring-system of the aryl or alkyl-aryl, cycloalkyl or alkyl-cycloalkyl; heterocyclyl or alkyl-heterocyclyl by OH, SH, =O, halogen (F, Cl, Br, I), CN, $NO_2$, COOH; $NR_xR_y$, with $R_x$ and $R_y$ independently being either H or a saturated or unsaturated, linear or branched, substituted or unsubstituted $C_{1-6}$-alkyl; a saturated or unsaturated, linear or branched, substituted or unsubstituted $C_{1-6}$-alkyl; a saturated or unsaturated, linear or branched, substituted or unsubstituted $-O-C_{1-6}$-alkyl (alkoxy); a saturated or unsaturated, linear or

branched, substituted or unsubstituted -S-$C_{1-6}$-alkyl; a saturated or unsaturated, linear or branched, substituted or unsubstituted - C(O)-$C_{1-6}$-alkyl-group; a saturated or unsaturated, linear or branched, substituted or unsubstituted -C(O)-O-$C_{1-6}$-alkyl-group; a substituted or unsubstituted aryl or alkyl-aryl; a substituted or unsubstituted cycloalkyl or alkyl-cycloalkyl; a substituted or unsubstituted heterocyclyl or alkyl-heterocyclyl.

The term "salt" is to be understood as meaning any form of the active compound used according to the invention in which it assumes an ionic form or is charged and is coupled with a counter-ion (a cation or anion) or is in solution. By this are also to be understood complexes of the active compound with other molecules and ions, in particular complexes which are complexed via ionic interactions.

The term "physiologically acceptable salt" means in the context of this invention any salt that is physiologically tolerated (most of the time meaning not being toxic-especially not caused by the counter-ion) if used appropriately for a treatment especially if used on or applied to humans and/or mammals.

These physiologically acceptable salts can be formed with cations or bases and in the context of this invention is understood as meaning salts of at least one of the compounds used according to the invention - usually a (deprotonated) acid - as an anion with at least one, preferably inorganic, cation which is physiologically tolerated - especially if used on humans and/or mammals. The salts of the alkali metals and alkaline earth metals are particularly preferred, and also those with NH4, but in particular (mono)- or (di)sodium, (mono)- or (di)potassium, magnesium or calcium salts.

These physiologically acceptable salts can also be formed with anions or acids in the context of this invention is understood as meaning salts of at least one of the compounds used according to the invention - usually protonated, for example on the nitrogen - as the cation with at least one anion which are physiologically tolerated - especially if used on humans and/or mammals. By this is understood in particular, in the context of this invention, the salt formed with a physiologically tolerated acid, that is to say salts of the particular active compound with inorganic or organic acids which are physiologically tolerated - especially if used on humans and/or mammals. Examples of physiologically tolerated salts of particular acids are salts of: hydrochloric acid, hydrobromic acid, sulfuric acid, methanesulfonic acid, formic acid, acetic acid, oxalic acid, succinic acid, malic acid, tartaric acid, mandelic acid, fumaric acid, lactic acid or citric acid.

The compounds of the invention may be in crystalline form or either as free compounds or as solvates and it is intended that those forms are within the scope of the present invention. Methods of solvation are generally known within the art. Suitable solvates are pharmaceutically acceptable solvates. The term "solvate" according to this invention is to be understood as meaning any form of the active compound according to the invention in which this compound has attached to it via non-covalent binding another molecule (most likely a polar solvent) especially including hydrates and alcoholates, e.g. methanolate.

Any compound that is a prodrug of a compound of formula (I) is within the scope of the invention. The term "prodrug" is used in its broadest sense and encompasses those Derivatives that are converted in vivo to the compounds of the invention. Such Derivatives would readily occur to those skilled in the art, and include, depending on the functional groups present in the molecule and without limitation, the following Derivatives of the present compounds: esters, amino acid esters, phosphate esters, metal salts sulfonate esters, carbamates, and amides. Examples of well known methods of producing a prodrug of a given acting compound are known to those skilled in the art and can be found e.g. in Krogsgaard-Larsen et al. "Textbook of Drug design and Discovery" Taylor & Francis (April 2002).

Unless otherwise stated, the compounds of the invention are also meant to include compounds which differ only in the presence of one or more isotopically enriched atoms. For example, compounds having the present structures except for the replacement of a hydrogen by a deuterium or tritium, or the replacement of a carbon by $^{13}C$- or $^{14}C$-enriched carbon or $^{15}N$-enriched nitrogen are within the scope of this invention.

The compounds of formula (I) or their salts or solvates are preferably in pharmaceutically acceptable or substantially pure form. By pharmaceutically acceptable form is meant, inter alia, having a pharmaceutically acceptable level of purity excluding normal pharmaceutical additives such as diluents and carriers, and including no material considered toxic at normal dosage levels. Purity levels for the drug substance are preferably above 50%, more preferably above 70%, most preferably above 90%. In a preferred embodiment it is above 95% of the compound of formula (I) or, or of its salts, solvates or prodrugs.

In a preferred embodiment of the compound according to the invention according to general formula (I) or (Ia) $R^1$ is selected from H; an optionally at least monosubstituted, linear or branched $C_{1-18}$-aliphatic group; an optionally at least monosubstituted alkyl-aryl; an optionally at least monosubstituted alkyl-heterocyclyl; or an optionally at least monosubstituted alkyl-cycloalkyl.

In another preferred embodiment of the compound according to the invention according to general formula (I) or (Ia) $R^2$ is selected from H; $(CH_2)_qCN$ with q being 0, 1, 2, 3 or 4; or OR with R being H or an optionally at least monosubstituted, linear or branched $C_{1-6}$-aliphatic group; preferably $R^2$ is selected from H; CN; $CH_2CN$; or OR with R being H or a linear or branched $C_{1-4}$-alkyl group, more preferably $R^2$ is selected from H, CN; $CH_2CN$; OH or $OCH_3$.

In another preferred embodiment of the compound according to the invention according to general formula (I) or (Ia) $R^3$ is selected from H; or an optionally at least monosubstituted, linear or branched $C_{1-6}$-aliphatic group; especially $R^3$ is selected from H; or an optionally at least monosubstituted, linear or branched $C_{1-6}$-alkyl group.

In another preferred embodiment of the compound according to the invention according to general formula (I) or (Ia) m and n are selected from 1 or 2 and m+n are selected from 3 or 4.

In another very preferred embodiment of the compound according to the invention the compounds are compounds according to general formula (Ib)

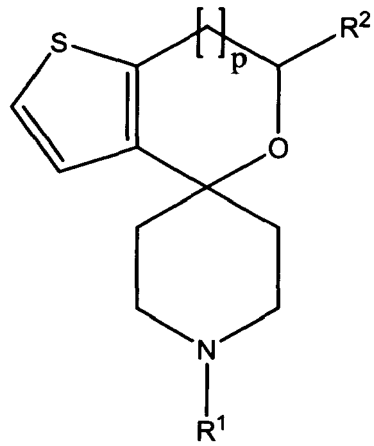

(Ib)

wherein

p is selected from 0 or 1;

$R^1$ is selected from hydrogen; an optionally at least monosubstituted, linear or branched $C_{1-18}$-aliphatic group; an optionally at least monosubstituted aryl; an optionally at least monosubstituted heterocyclyl; an optionally at least monosubstituted cycloalkyl; an optionally at least monosubstituted alkyl-aryl; an optionally at least monosubstituted alkyl-heterocyclyl; an optionally at least monosubstituted alkyl-cycloalkyl; or COOR' with R' being either H or $C_{1-4}$-alkyl;

$R^2$ is selected from hydrogen; an optionally at least monosubstituted, linear or branched $C_{1-6}$-aliphatic group; $(CH_2)_qCN$ with q being 0, or 1; or OR with R being H or an optionally at least monosubstituted, linear or branched $C_{1-6}$-aliphatic group;

optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another preferred embodiment of the compound according to the invention according to general formula (Ib) $R^2$ is selected from H; $CH_2CN$; CN; or OR with R being H or an optionally at least monosubstituted, linear or branched $C_{1-6}$-alkyl group; especially $R^2$ is selected from H; $CH_2CN$; CN; OH or a linear or branched $OC_{1-4}$-alkyl group; more especially $R^2$ is selected from H, CN, $CH_2CN$, OH or $OCH_3$.

In another preferred embodiment of the compound according to the invention according to general formula (Ib) $R^1$ is selected from hydrogen; an optionally at least monosubstituted, linear or branched $C_{1-18}$-aliphatic group; an optionally at least monosubstituted alkyl-aryl; an optionally at least monosubstituted alkyl-heterocyclyl; or an optionally at least monosubstituted alkyl-cycloalkyl.

In another preferred embodiment of the compound according to the invention according to general formula (Ib) $R^1$ is selected from

hydrogen, $C_{1-10}$-alkyl, linear or branched; $C_{1-10}$-alkenyl, linear or branched; optionally at least mono-substituted $C_{1-6}$-alkyl-aryl; optionally at least mono-substituted $C_{1-6}$-alkyl-heterocyclyl; or optionally at least mono-substituted $C_{1-6}$-alkyl-cycloalkyl;

preferably $R^1$ is selected from hydrogen; $C_{1-10}$-alkyl, linear or branched; $C_{2-8}$-alkenyl, linear or branched; optionally

at least mono-substituted $C_{1-6}$-alkyl-aryl; optionally at least mono-substituted $C_{1-6}$-alkyl-heterocyclyl; or optionally at least mono-substituted $C_{1-6}$-alkyl-$C_{4-8}$-cycloalkyl.

In another highly preferred embodiment of the compound according to the invention the compounds are compounds according to general formula (Ib)

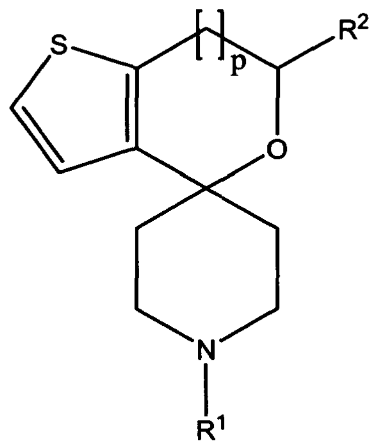

(Ib)

wherein

p is selected from 0 or 1;

$R^1$ is selected from hydrogen; $C_{1-10}$-alkyl, linear or branched; $C_{2-8}$-alkenyl, linear or branched; optionally at least mono-substituted $C_{1-6}$-alkyl-aryl; optionally at least mono-substituted $C_{1-6}$-alkyl-heterocyclyl; or optionally at least mono-substituted $C_{1-6}$-alkyl-$C_{4-8}$-cycloalkyl;

$R^2$ is selected from H; CN; $CH_2CN$; OH or a linear or branched $OC_{1-4}$-alkyl group;

optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another preferred embodiment of the compound according to the invention the compounds according to general formula (I) or (Ia) are selected from:

- Ethyl-6'-methoxy-6',7'-dihydrospiro[piperidin-4,4'-thieno[3,2-c]pyran]-1-carboxylate;
- 6'-Methoxy-6',7'-dihydrospiro[piperidin-4,4'-thieno[3,2-c]pyran;
- 1-Benzyl-6'-methoxy-6',7'-dihydrospiro[piperidin-4,4'-thieno[3.2-c]pyran];
- 6'-Methoxy-1-(2-phenylethyl)-6',7'-dihydrospiro[piperidin-4,4'-thieno[3.2-c]pyran];
- 1-(Cyclohexylmethyl)-6'-Methoxy -6',7'-dihydrospiro[piperidin-4,4'-thieno[3.2-c]pyran];
- 6'-Methoxy-1-(3-methylbutyl)-6',7'-dihydrospiro[piperidin-4,4'-thieno[3.2-c]pyran];
- 6'-Methoxy-1-(3-methylbut-2-enyl)-6',7'-dihydrospiro[piperidin-4,4'-thieno[3.2-c]pyran];
- 1-Butyl-6'-methoxy-6',7'-dihydrospiro[piperidin-4,4'-thieno[3.2-c]pyran];
- 6'-Methoxy-1-pentyl-6',7'-dihydrospiro[piperidin-4,4'-thieno[3.2-c]pyran];
- 6'-Methoxy-1-octyl -6',7'-dihydrospiro[piperidin-4,4'-thieno[3.2-c]pyran];
- 3'-Methoxy-1-(3-phenylpropyl)-6',7'-dihydrospiro[piperidin-4,4'-thieno[3.2-c]pyran];
- 3'-Methoxy-1-(4-phenylbutyl)-6',7'-dihydrospiro[piperidin-4,4'-thieno[3.2-c]pyran];
- 1-(4-Fluorbenzyl)-6'-Methoxy -6',7'-dihydrospiro[piperidin-4,4'-thieno[3.2-c]pyran];
- 6'-Methoxy-1-(4-methoxybenzyl)-6',7'-dihydrospiro[piperidin-4,4'-thieno[3.2-c]pyran];
- 6'-Methoxy-1-(thien-2-ylmethyl)-6',7'-dihydrospiro[piperidin-4,4'-thieno[3.2-c]pyran];
- 1-Benzylspiro[piperidin-4,4'-thieno[3,2-c]pyran];

- 1-Benzyl-6',7'-dihydrospiro[piperidin-4,4'-thieno[3,2-c]pyran]-6'-carbonitrile;
- {1-Benzyl-6',7'-dihyrospiro[piperidin-4,4'-thieno[3,2-c]pyran]-6'-yl}acetonitrile;
- 1-Benzyl-6',7'-dihydrospiro[piperidin-4,4'-thieno[3,2-c]pyran]; or
- 1-Benzyl-6',7'-dihydrospiro[piperidin-4,4'-thieno[3,2-c]pyran]-6'-ol;

optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof;
preferably from

- 1-Benzyl-6'-methoxy-6',7'-dihydrospiro[piperidin-4,4'-thieno[3.2-c]pyran];
- 6'-Methoxy-1-(2-phenylethyl)-6',7'-dihydrospiro[piperidin-4,4'-thieno[3.2-c]pyran];
- 1-(Cyclohexylmethyl)-6'-Methoxy -6',7'-dihydrospiro[piperidin-4,4'-thieno[3.2-c]pyran];
- 6'-Methoxy-1-(3-methylbutyl)-6',7'-dihydrospiro[piperidin-4,4'-thieno[3,2-c]pyran];
- 6'-Methoxy-1-(3-methylbut-2-enyl)-6',7'-dihydrospiro[piperidin-4,4'-thieno[3.2-c]pyran];
- 1-Butyl-6'-methoxy-6',7'-dihydrospiro[piperidin-4,4'-thieno[3.2-c]pyran];
- 6'-Methoxy-1-pentyl-6',7'-dihydrospiro[piperidin-4,4'-thieno[3.2-c]pyran];
- 6'-Methoxy-1-octyl -6',7'-dihydrospiro[piperidin-4,4'-thieno[3.2-c]pyran];
- 3'-Methoxy-1-(3-phenylpropyl)-6',7'-dihydrospiro[piperidin-4,4'-thieno[3.2-c]pyran];
- 3'-Methoxy-1-(4-phenylbutyl)-6',7'-dihydrospiro[piperidin-4,4'-thieno[3.2-c]pyran];
- 1-(4-Fluorbenzyl)-6'-Methoxy -6',7'-dihydrospiro[piperidin-4,4'-thieno[3.2-c]pyran];
- 6'-Methoxy-1-(4-methoxybenzyl)-6',7'-dihydrospiro[piperidin-4,4'-thieno[3.2-c]pyran];
- 6'-Methoxy-1-(thien-2-ylmethyl)-6',7'-dihydrospiro[piperidin-4,4'-thieno[3.2-c]pyran];
- 1-Benzyl-6',7'-dihydrospiro[piperidin-4,4'-thieno[3,2-c]pyran]-6'-carbonitrile;
- {1-Benzyl-6',7'-dihyrospiro[piperidin-4,4'-thieno[3,2-c]pyran]-6'-yl}acetonitrile;
- 1-Benzyl-6',7'-dihydrospiro[piperidin-4,4'-thieno[3,2-c]pyran]; or
- 1-Benzyl-6',7'-dihydrospiro[piperidin-4,4'-thieno[3,2-c]pyran]-6'-ol;

optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

The term "pharmacological tool" refers to the property of compounds of the invention through which they are particularly selective ligands for Sigma receptors which implies that compound of formula (I), described in this invention, can be used as a model for testing other compounds as sigma ligands, ex. a radiactive ligands being replaced, and can also be used for modeling physiological actions related to sigma receptors.

The compounds of the present invention represented by the above described formula (I) may include enantiomers depending on the presence of chiral centres or isomers depending on the presence of multiple bonds (e.g. Z, E). The single isomers, enantiomers or diastereoisomers and mixtures thereof fall within the scope of the present invention.

In general the processes are described below in the experimental part. The starting materials are commercially available or can be prepared by conventional methods.

A preferred aspect of the invention is also a process for the production of a compound according to the invention according to general formula (I), wherein a compound of formula (III)

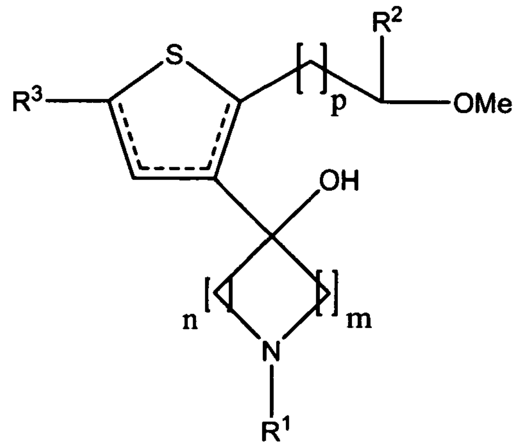

(III)

, wherein $R^1$, $R^2$, $R^3$, m, n and p are as defined above, is reacted with p-toluol sulphonic acid to form acompound according the formula I. It is preferred if in this process

- $R^2$ is $OCH_3$; and/or
- $R^3$ is H and/or
- p is H.

A preferred aspect of the invention is also a process for the production of a compound according to the invention according to general formula (Ib), wherein a compound of formula (IIIb)

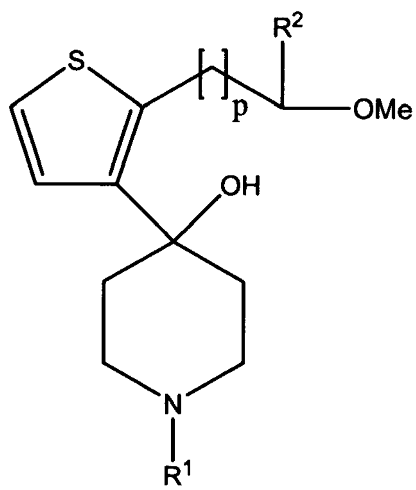

(IIIb)

, wherein $R^1$, $R^2$ and p are as defined above is reacted with p-toluol sulphonic acid to form acompound according the formula (Ib). It is preferred if

- $R^2$ is $OCH_3$ and/or

- pis 1.

It is further preferred if in the process above as a next step a compound according to formula (I) or (Ib) in which $R^3$ is C(O)OR' is reacted with a alkaline solution in water to form a compound according to formulas (I) or (Ib) with $R^1$ being H.

The obtained reaction products may, if desired, be purified by conventional methods, such as crystallisation and chromatography. Where the above described processes for the preparation of compounds of the invention give rise to mixtures of stereoisomers, these isomers may be separated by conventional techniques such as preparative chromatography. If there are chiral centers the compounds may be prepared in racemic form, or individual enantiomers may be prepared either by enantiospecific synthesis or by resolution.

One preferred pharmaceutically acceptable form is the crystalline form, including such form in pharmaceutical composition. In the case of salts and solvates the additional ionic and solvent moieties must also be non-toxic. The compounds of the invention may present different polymorphic forms, it is intended that the invention encompasses all such forms.

Another aspect of the invention refers to a pharmaceutical composition which comprises a compound according to the invention or a pharmaceutically acceptable salt, prodrug, isomer or solvate thereof, and a pharmaceutically acceptable carrier, adjuvant or vehicle. The present invention thus provides pharmaceutical compositions comprising a compound of this invention, or a pharmaceutically acceptable salt, derivative, prodrug or stereoisomers thereof together with a pharmaceutically acceptable carrier, adjuvant, or vehicle, for administration to a patient.

Examples of pharmaceutical compositions include any solid (tablets, pills, capsules, granules etc.) or liquid (solutions, suspensions or emulsions) composition for oral, topical or parenteral administration.

In a preferred embodiment the pharmaceutical compositions are in oral form, either solid or liquid. Suitable dose forms for oral administration may be tablets, capsules, syrops or solutions and may contain conventional excipients known in the art such as binding agents, for example syrup, acacia, gelatin, sorbitol, tragacanth, or polyvinylpyrrolidone; fillers, for example lactose, sugar, maize starch, calcium phosphate, sorbitol or glycine; tabletting lubricants, for example magnesium stearate; disintegrants, for example starch, polyvinylpyrrolidone, sodium starch glycollate or microcrystalline cellulose; or pharmaceutically acceptable wetting agents such as sodium lauryl sulfate.

The solid oral compositions may be prepared by conventional methods of blending, filling or tabletting. Repeated blending operations may be used to distribute the active agent throughout those compositions employing large quantities of fillers. Such operations are conventional in the art. The tablets may for example be prepared by wet or dry granulation and optionally coated according to methods well known in normal pharmaceutical practice, in particular with an enteric coating.

The pharmaceutical compositions may also be adapted for parenteral administration, such as sterile solutions, suspensions or lyophilized products in the apropriate unit dosage form. Adequate excipients can be used, such as bulking agents, buffering agents or surfactants.

The mentioned formulations will be prepared using standard methods such as those described or referred to in the Spanish and US Pharmacopoeias and similar reference texts.

Administration of the compounds or compositions of the present invention may be by any suitable method, such as intravenous infusion, oral preparations, and intraperitoneal and intravenous administration. Oral administration is preferred because of the convenience for the patient and the chronic character of the diseases to be treated.

Generally an effective administered amount of a compound of the invention will depend on the relative efficacy of the compound chosen, the severity of the disorder being treated and the weight of the sufferer. However, active compounds will typically be administered once or more times a day for example 1, 2, 3 or 4 times daily, with typical total daily doses in the range of from 0.1 to 1000 mg/kg/day.

The compounds and compositions of this invention may be used with other drugs to provide a combination therapy. The other drugs may form part of the same composition, or be provided as a separate composition for administration at the same time or at different time.

Another aspect of the invention refers to the use of a compound according to the invention in the manufacture of a medicament.

Another aspect of the invention refers to the use of a compound according to the invention in the manufacture of a medicament for the treatment or prophylaxis of a sigma receptor mediated disease or condition. A preferred embodiment of this is this use wherein the disease is diarrhoea, lipoprotein disorders, metabolic syndrome, treatment of elevated triglyceride levels, chylomicronemia, hyperlipoproteinemia; hyperlipidemia, especially mixed hyperlipidemia; hypercholesterolemia, dysbetalipoproteinemia, hypertriglyceridemia including both the sporadic and familial disorder (inherited hypertriglyceridemia), migraine, obesity, arthritis, hypertension, arrhythmia, ulcer, learning, memory and attention deficits, cognition disorders, neurodegenerative diseases, demyelinating diseases, addiction to drugs and chemical substances including cocaine, amphetamine, ethanol and nicotine, tardive diskinesia, ischemic stroke, epilepsy, stroke, depression, stress, psychotic condition, schizophrenia; inflammation, autoimmune diseases or cancer.

A preferred embodiment of this is this use wherein the disease is pain, especially neuropathic pain, inflammatory

pain or other pain conditions, allodynia and/or hyperalgesia, especially mechanical allodynia.
Another aspect of the invention refers to the use of a compound according o the invention as pharmacological tool or as anxiolytic or immunosuppressant.

The term "pharmacological tool" refers to the property of compounds of the invention through which they are particularly selective ligands for Sigma receptors which implies that compound of formula I, described in this invention, can be used as a model for testing other compounds as Sigma ligands, ex. a radiactive ligands being replaced, and can also be used for modeling physiological actions related to Sigma receptors.

Another aspect of this invention relates to a method of treating or preventing a sigma receptor mediated disease which method comprises administering to a patient in need of such a treatment a therapeutically effective amount of a compound as above defined or a pharmaceutical composition thereof. Among the sigma mediated diseases that can be treated are diarrhoea, lipoprotein disorders, metabolic syndrome, treatment of elevated triglyceride levels, chylomicronemia, hyperlipoproteinemia; hyperlipidemia, especially mixed hyperlipidemia; hypercholesterolemia, dysbetalipoproteinemia, hypertriglyceridemia including both the sporadic and familial disorder (inherited hypertriglyceridemia), migraine, obesity, arthritis, hypertension, arrhythmia, ulcer, learning, memory and attention deficits, cognition disorders, neurodegenerative diseases, demyelinating diseases, addiction to drugs and chemical substances including cocaine, amphetamine, ethanol and nicotine, tardive diskinesia, ischemic stroke, epilepsy, stroke, depression, stress, pain, especially neuropathic pain, inflammatory pain or other pain conditions, allodynia and/or hyperalgesia, especially mechanical allodynia, psychotic condition, schizophrenia; inflammation, autoimmune diseases or cancer; disorders of food ingestion, the regulation of appetite, for the reduction, increase or maintenance of body weight, for the prophylaxis and/or treatment of obesity, bulimia, anorexia, cachexia or type II diabetes, preferably type II diabetes caused by obesity. The compounds of the invention can also be employed as pharmacological tool or as anxiolytic or immunosuppressant.

The compounds of the invention may be synthesized following one of the Reaction Schemes (A, or B) set out below:

**Reaction Scheme A:** (with EX meaning „Example")

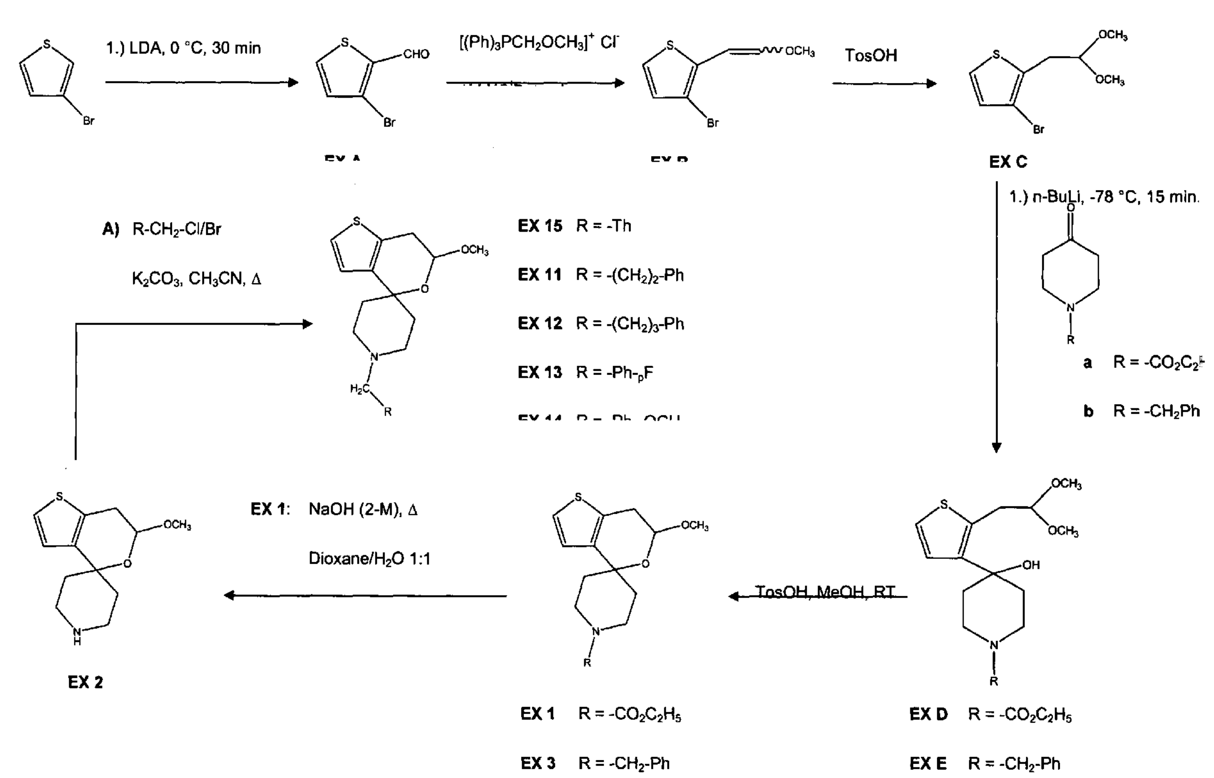

**Reaction Scheme B:** (with EX meaning „Example")

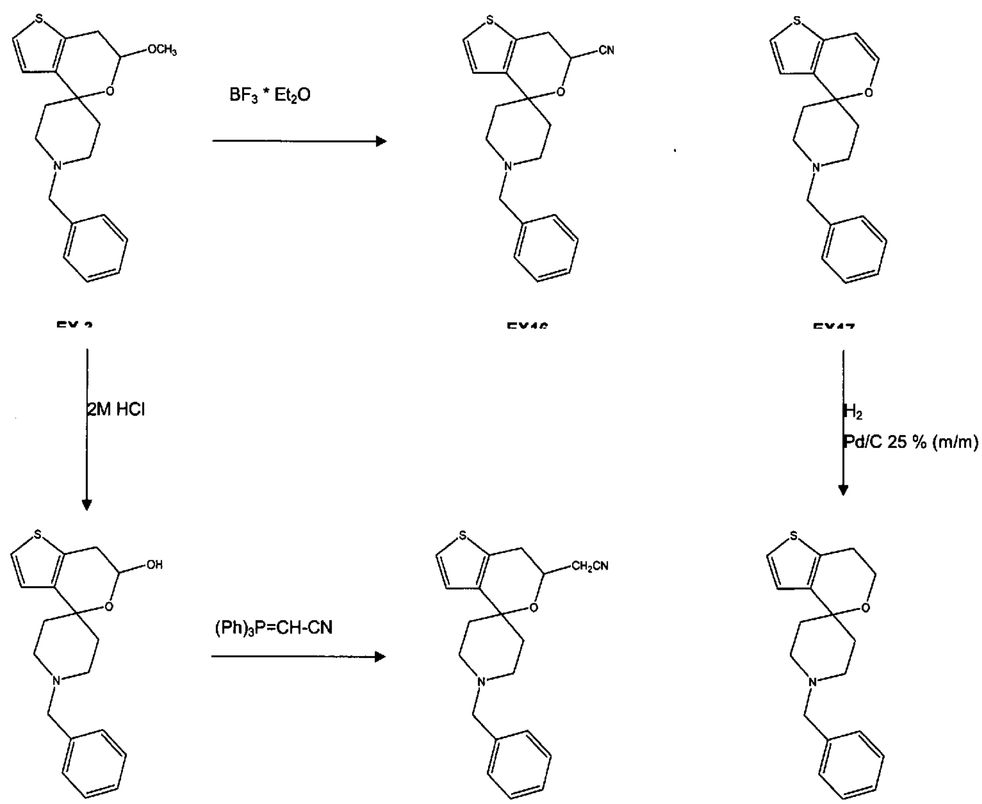


The present invention is illustrated below with the aid of examples. These illustrations are given solely by way of example and do not limit the general spirit of the present invention.

**EXAMPLES:**

**General Experimental Part (Methods and Equipment of the synthesis and analysis**

All solvents used for synthesis were p. a. quality.

The separation of mixtures of substances using thin-layer chromatography was done on glass plates layered by silica gel. Analysis of the plates was done after treatment with iodine gas or incubation with Dragendorffs reagent under UV light.

As a rule synthesized products were purified using flash-chromatography.

Melting points were measured using capillaries. As sometimes the products were mixtures of diastereoisomers, themelting point: had to be expressed as a range.

IR-spectra were measured using the FT-IR-480 Plus Fourier Transform Spectrometer with ATR (Fa. Jasco). All substances were either measured directly as solids or in oil.

NMR-Spectra were measured using Mercury-400BB (Fa. Varian) at a temperature of 21 °C. δ, measured in ppm, is based on the signal TMS measured in comparison to the residue signal ($CHCl_3$) of the solvent ($CDCl_3$):

$^1$H-NMR-Spectroscopy:

$$\delta\,(\mathrm{TMS}) = \delta\,(\mathrm{CHCl_3}) - 7.26$$

$^{13}$C-NMR-Spectroscopy:

$$\delta\ (TMS) = \delta\ (CHCl_3) - 77.0$$

Mass-spectra (MS) were measured using the GCQ Finnigan MAT (Fa. Finnigan) with Xcalibur Version 1.1. The method of ionisation is shown in brackets: EI = electronic ionisation (70 eV); CI = Chemical ionisation (Isobutane or $NH_3$, 170 eV).
Elementary analysis was done with VarioEL (Fa. Elementar).

Before using HPLC the maximum absorption of the substances was measured using the UV/Vis-Spectra done with a 50Bio Cary Spektrophotometer (Fa. Varian).
For determination of the purity and for the separation of the diastereomers a HPLC Hitachi L6200A Intelligent Pump with a UV-Detector (Merck) was used.
Synthesis was done in the synthetic microwave Discover (Fa. CEM).
Some reactions were done using protective gas.
Reactions at -78˚C were done in an acetone bath in a Dewar.

**Example A: 3-Bromthiophen-2-carbaldehyd**

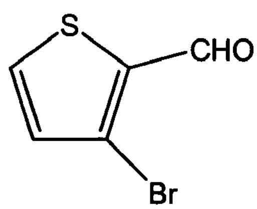

Experimental procedure

3-Bromo-thiophene (6.523 g, 40 mmol) was dissolved under $N_2$ in THF (100 mL). After cooling to 0-2 ˚C it was mixed dropwise with a 2M LDA solution (20 mL, 40 mmol) and stirred for 0.5 h. Following that 1-Formylpiperidin (4.52 g, 40 mmol) was added. After 2 h the reaction was stopped by adding a surplus of a saturated solution of $NH_4Cl$. For purification it was extracted three times with $Et_2O$ (10 mL) and the pooled organic phases subsequently dried over $Na_2SO_4$. After filtration the solvent was removed under vacuum. The crude product (approx. 8.3 g) was purified using flash-chromatography (8 cm, cyclohexane:ethylacetate 9:1, 30 mL, $R_f$ = 0.65) and by distillation under vacuum (Bp. 62˚C, $5.6 \cdot 10^{-2}$ mbar).
Colourless liquid, yield 6.05 g (79.2 %).
$C_5H_3BrOS$ (191.1)
MS (EI): m/z = 190/192 [$M^+$], 162 [$M^+$ -CHO].
IR (Film): ν ($cm^{-1}$) = 3103 (C-H), 2843 (C-H), 1657 (C=O).
$^1H$-NMR ($CDCl_3$):

δ (ppm) = 7.15 (d, J = 5.5 Hz, 1 H, 4-*H*-Th), 7.71 (dd, J = 5.1/1.5 Hz, 1 H, 5-H-Th), 9.99 (d, J = 1.5 Hz, 1 H, Th-CHO).

**Example B: cis/trans-3-Brom-2-(2-methoxyvinyl)-thiophene**

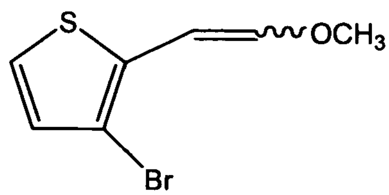

Experimental procedure

Methoxymethyltriphenylphosphoniumchloride (13.1 g, 38.3 mmol) was suspended in a 500 mL flask under $N_2$ in THF (200 ml). After cooling to T < -10 °C it was mixed with a 1 M solution of potassium tertiary butylate (38.3 mL, 38.3 mmol) (change of colour to red). Few minutes later Example A (5.64 g, 29.5 mmol) was added (change of colour to yellow). After 2 h of reaction time at -10 °C purification followed. Thus, $H_2O$ (80 mL) was added and three times extracted with $Et_2O$ (15 mL). The pooled organic phases were dried over $Na_2SO_4$ and subsequently filtered. Then the solvent was removed under vacuum. Triphenylphosphaneoxide was separated from the crude product by extracting with a mixture of cyclohexane/ethylacetate. The crude product was purified by distillation under vacuum (Bp. 72 °C 5.6 $\cdot 10^{-2}$ mbar).

Colourless liquid, yield : 5.39 g (83 %).

$C_7H_7BrOS$ (219.1)

MS (EI): m/z = 218/220 [$M^+$], 204 [$M^+$ -$CH_3$], 175 ($M^+$ - $CH(OCH_3)$)].

IR (Film): v ($cm^{-1}$) = 3008 (C=C-H), 2833 (C-H), 1634 (C=C), 1225 (C=C-O).

**cis-3-Brom-2-(2-methoxyvinyl)-thiophene**

$^1$H-NMR ($CDCl_3$):

δ (ppm) = 3.77 (s, 3H, $OCH_3$), 5.70 (dd, J = 6.6/1.9 Hz, 1 H, Th-C*H*=$CHOCH_3$), 6.18 (d, J = 6.6 Hz, 1 H, Th-CH=C*H*$OCH_3$), 6.85 (d, J = 5.4 Hz, 1 H, 4-*H*-Th), 7.09 (dd, J = 5.4/0.9 Hz, 1 H, 5-*H*-Th).

**trans3-Brom-2-(2-methoxyvinyl)-thiophene**

$^1$H-NMR ($CDCl_3$):

δ (ppm) = 3.64 (s, 3 H, $OCH_3$), 5.89 (d, J = 12.9 Hz, 1 H, Th-C*H*=$CH(OCH_3)$), 6.82 (d, J = 5.4 Hz, 1 H, 4-H-Th), 6.89 (d, J = 5.4 Hz, 1 H, 5-H-Th), 6.97 (d, J = 12.9 Hz, 1 H, Th-CH=C*H*$OCH_3$).

Cis- und trans- are occurring in a ratio of 2:1.

**Example C: 2-(3-Bromthiophen-2-yl)-acetaldehyddimethylacetal**

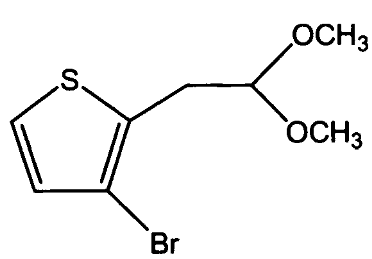

Experimental procedure

Example B (5.39 g, 24.6 mmol) was dissolved under $N_2$ in 80 mL MeOH. After addition of 10 mol% of p-toluene sulphonic acid (470 mg, 2.46 mmol) and trimethylorthoformiate (5 mL) it was heated for 24 h under reflux. For purification

it was alkalised with 2M NaOH (15 mL) (pH = 10) and $H_2O$ (30 mL) added and then extracted three times with $CH_2Cl_2$ (10 mL). The pooled organic phases were dried over $Na_2SO_4$ and subsequently filtered. The solvent was removed under vacuum. Purification of crude product (approx. 5.8 g) was done by distillation under vacuum (Bp. 78 ˚C, $5.6 \cdot 10^{-2}$ mbar).

Colourless liquid, yield 5.73 g (93 %).

$C_8H_{11}BrO_2S$ (251.1)

MS (EI): m/z = 251 [$M^+$], 220 [$M^+$ -$OCH_3$], 140 [$M^+$ -Br, -$OCH_3$].

IR (Film): ν ($cm^{-1}$) = 3106 (C-H), 2830 (C-H), 1057 (C-O).

$^1$H-NMR ($CDCl_3$):

δ (ppm) = 3.03 (d, J = 5.6 Hz, 2 H, Th-C$H_2$CH($OCH_3$)$_2$), 3.31 (s, 6 H, Th-$CH_2$CH(OC$H_3$)$_2$), 4.47 (t, J = 5.6 Hz, 1 H, Th-$CH_2$C*H*($OCH_3$)$_2$), 6.84 (d, J = 5.4 Hz, 1 H, 4-H-Th), 7.15 (d, J = 5.4 Hz, 1 H, 5-H-Th).

Example D: Ethyl-4-[2-(2,2-dimethoxyethyl)-thiophen-3-yl]-4-hydroxypiperidin-1-carboxylat

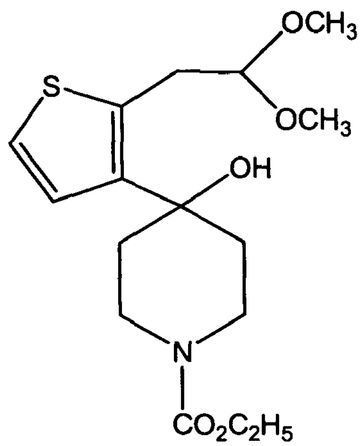

Experimental procedure

Example A (1.52 g, 6 mmol) was dissolved under $N_2$ in THF (80 mL) and cooled to - 80 ˚C. A solution of 1.35M n-butyllithium in n-hexane (5.2 mL, 7.8 mmol) was added dropwise within 2-3 min and stirred for 15 min. Following that 1-ethoxycarbonylypiperidin-4-one (1.02 g, 6.0 mmol), dissolved in THF (1.5 mL), was added and it was first stirred for one hour at -80 ˚C and subsequently for 3 h at RT. For purification $H_2O$ (30 mL) was added and extracted three times with 10 mL $CH_2Cl_2$ extracted. The pooled organic phases were dried over $Na_2SO_4$ und subsequently filtered. The solvent was removed under vacuum. The crude product (approx. 2.22 g) was purified using flash-chromatography (4 cm, Cyclohexane:ethylacetate 7:3, 30 mL, $R_f$ = 0.3).

Yellowish oil, yield 1.48 g (approx. 70 %, slightly polluted with some 1-ethoxy-carbonylpiperidin-4-one).

$C_{16}H_{25}NO_5S$ (343.4)

MS (EI): m/z = 343 [$M^+$], 264 [$M^+$ -OH, -$OCH_3$ (2x)], 210 [$M^+$ -$OCH_3$ (2x), -$COOC_2H_5$].

IR (Film): ν ($cm^{-1}$) = 3436 (O-H), 2843 (C-H), 1671 (C=O), 1051 (C-O).

$^1$H-NMR ($CDCl_3$):

δ (ppm) = 1.25 (t, J = 7.2 Hz, 3H, $CO_2CH_2$C$H_3$), 1.65 (d, J = 14.4 Hz, 2 H, N($CH_2$C$H_2$)$_2$), 1.87 (t, J = 14.4 Hz, 2 H, N($CH_2$C$H_2$)$_2$), 3.09 - 3.25 (m, 4 H, (Th-C$H_2$CH) (N(C$H_2CH_2$)$_2$)), 3.32 (s, 6H, CH(OC$H_3$)$_2$), 3.90 - 4.03 (m, 2H, N (C$H_2CH_2$)$_2$), 4.07 (q, J = 7.2 Hz, 2 H, $CO_2$C$H_2CH_3$), 4.43 (t, J = 5.2 Hz, 1 H, Th-$CH_2$C*H*), 6.73 (d, J = 5.6 Hz, 1 H, 4-H-Th), 7.11 (d, J = 5.2 Hz, 1 H, 5-*H*-Th).

**Example 1: Ethyl-6'-methoxy-6',7'-dihvdrospiro[piperidine-4,4'-thieno[3,2-c]pyran]-1-carboxylate**

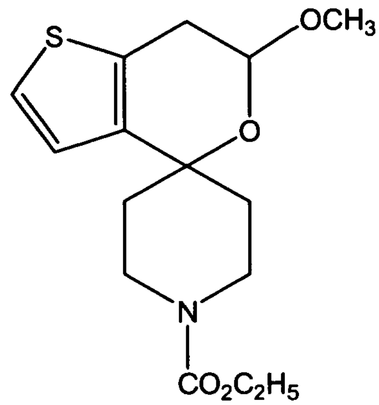

Experimental procedure

Example D (1.49 g, 4.3 mmol) was dissolved under $N_2$ in MeOH (50 mL). After addition of 1.2 equivalents of p-toluene sulphonic acid (1 g, 5.2 mmol) and trimethylorthoformiate (5 mL) it was stirred for 1 h at RT. For purification it was alkalised with 2M NaOH and $H_2O$ (30 mL) added. Following that it was extracted three times with $CH_2Cl_2$ (10 mL). The pooled organic phases were dried over Na2SO4 und subsequently filtered. The solvent was removed under vacuum. The crude product (approx. 1.56 g) was purified using flash-chromatography (3 cm, cyclohexane:ethylacetate 7:3, 30 mL, $R_f$ = 0.54).

Colourless oil, yield 990 mg (53 % over two steps).

$C_{15}H_{21}NO_4S$ (311.4)

MS (EI): m/z = 311 [$M^+$], 280 [$M^+$ -$OCH_3$].

IR (Film): ν ($cm^{-1}$) = 2924 (C-H), 1693 (C=O), 1059 (C-O), 718 (C-H).

$^1$H-NMR ($CDCl_3$):

δ (ppm) = 1.22 (t, J = 7.2 Hz, 3 H, $CO_2CH_2CH_3$), 1.69 (td, J = 13.5/4.6 Hz, 1 H, $N(CH_2CH_2)_2$), 1.76 (dd, J = 13.5/4.6 Hz, 1 H $N(CH_2CH)_2$), 1.83 (td, J = 13.5/4.6 Hz, 1 H, $N(CH_2CH_2)_2$), 1.89 (dd, J = 13.5/4.6 Hz, 1 H, $N(CH_2CH_2)_2$), 2.80 (dd, J = 15.9/7.2 Hz, 1 H, Th-$CH_2$), 2.95 (dd, J = 15.9/3.3 Hz, 1 H, Th-$CH_2$), 3.10 - 3.25 (m, 2H, $N(CH_2CH_2)_2$), 3.50 (s, 3 H, $OCH_3$), 3.98 - 4.15 (m, 2 H, $N(CH_2CH_2)_2$), 4.10 (q, J = 7.2 Hz, 2 H, $CO_2CH_2CH_3$), 4.83 (dd, J = 7.2/3.3 Hz, 1 H, Th-$CH_2CH$), 6.65 (d, J = 5.1 Hz, 1 H, 4-H-Th), 7.05 (d, J = 5.1 Hz, 1 H, 5-H-Th).

**Example 2: 6'-Methoxy-6',7'-dihydrospiro[piperidin-4,4'-thieno[3,2-c]pyran**

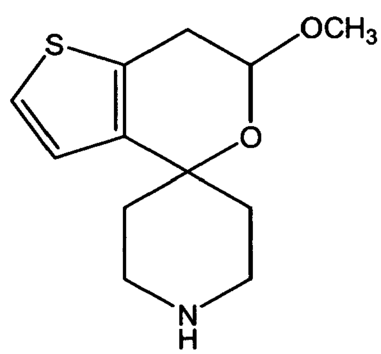

Experimental procedure

Example 1 (0.92 g, 2.96 mmol) was dissolved in a 100 mL flask in dioxane/$H_2O$ 1:1 (50 mL). After addition of 70 equivalents of 2M NaOH (100 mL) it was heated for 5 h under reflux. For purification $Et_2O$ (20 mL) was added and

again extracted three times with $Et_2O$ (5 mL). The pooled organic phases were dried over $Na_2SO_4$. Following filtration the solvent was removed under vacuum. The crude product (approx. 0.72 g) was purified using flash-chromatography (3 cm, MeOH:$NH_3$ 98:2, 15 mL, $R_f$ = 0.25).

yield: Colourless Solid , Mp. 103 ˚C, yield 0.385 g (54 %).

$C_{12}H_{17}NO_2S$ (239.3)

MS (EI): m/z = 239 [$M^+$], 208 [$M^+$ -$OCH_3$], 151 ($M^+$ -$OCH_3$, -$CH_2NHCH_2CH_2$).

IR (Film): v ($cm^{-1}$) = 3276 (N-H), 2915 (C-H), 1057 (C-O), 746 (C-H).

$^1$H-NMR ($CDCl_3$):

δ (ppm) = 1.68 (td, J = 14.0/4.7 Hz, 1 H, N($CH_2CH_2$)$_2$), 1.76 (dd, J = 14.0/2.7 Hz, 1 H, N($CH_2$C*H*$_2$)$_2$), 1.84 - 1.96 (m, 2 H, N($CH_2CH_2$)$_2$), 2.78 (dd, J = 15.6/7.5 Hz, 1 H, Th-C*H*$_2$-$CHOCH_3$), 2.84 - 2.88 (m, 1 H, N(C*H*$_2$$CH_2$)$_2$), 2.88 - 2.93 (m, 1 H, N(C*H*$_2$$CH_2$)$_2$), 2.92 (dd, J = 15.6/3.3 Hz, 1 H, Th-C*H*$_2$ $CHOCH_3$), 3.04 (td, J = 12.3/2.7 Hz, 1 H, N (C*H*$_2$$CH_2$)$_2$), 3.15 (td, J = 12.3/3.0 Hz, 1 H, N($CH_2CH_2$)$_2$), 3.52 (s, 3 H, Th-$CH_2$-CHOC*H*$_3$), 4.83 (dd, J = 7.2/3.3 Hz, 1 H, Th-$CH_2$-C*H*$OCH_3$), 6.72 (d, J = 5.1 Hz, 1 H, 4-H-Th), 7.03 (d, J = 5.1 Hz, 1 H, 5-H-Th). Ein Signal für NH ist nicht zu sehen.

Example E: 2-[3-(1-Benzyl-4-hydroxypiperidin-4-yl)thiophen-2-yl]acetaldehyddimethylacetal

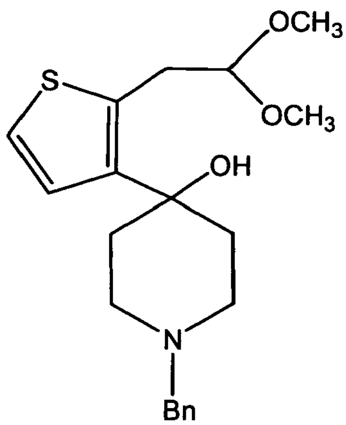

Experimental procedure

Example C (2.18 g, 8.7 mmol) was dissolved in THF (100 mL) and cooled under $N_2$ to -84 ˚C. A solution of 1,35M in n-butyllithium in hexane (8.3 mL, 11.3 mmol) was added dropwise within 2-3 min under stirring, until after 15 min 1-benzyl piperidin-4-one (1.81 g, 9.57 mmol) was added. After 1 h of stirring at -84 ˚C a further 2 h it was stirred at RT. The reaction was stopped by adding 20 mL of water. Then a solution of $NaHSO_3$ (10 mL, 10%.) was added and the mixture extracted three times with 10 mL of $CH_2Cl_2$. The organic phases were pooled and dried over $Na_2SO_4$, followed by filtration and the solvent was removed under vacuum. The crude product (approx. 3.31 g) was purified using flash-chromatography (5 cm, cyclohexane:ethylacetate 7:3, 30 mL, $R_f$= 0.3).

Yellowish oil, yield 2.14 g (68 %).

$C_{20}H_{27}NO_3S$ (361.5)

MS (EI): m/z = 361 [$M^+$], 330 [$M^+$-$OCH_3$], 270 [$M^+$ - $CH_2Ph$], 91 [-$CH_2Ph$].

IR (Film): v ($cm^{-1}$) = 3450 (O-H), 2851 (C-H), 1051 (C-O), 698 (C-H).

$^1$H-NMR ($CDCl_3$):

δ (ppm) = 1.67 (dd, J = 14.0/2.5 Hz, 2 H, N($CH_2$C*H*$_2$)$_2$), 2.04 (td, J = 13.2/4.5 Hz, 2 H, N($CH_2$C*H*$_2$)$_2$), 2.45 (td, J = 12.3/2.1 Hz, 2 H, N(C*H*$_2$$CH_2$)$_2$), 2.62 - 2.71 (m, 2 H, N(C*H*$_2$$CH_2$)$_2$), 3.30 (s, 6 H, Th-$CH_2$-CH(OC*H*$_3$)$_2$), 3.38 (d, J = 5.4 Hz, 2 H, Th-C*H*$_2$-CH($OCH_3$)$_2$), 3.50 (s, 2 H, N- C*H*$_2$-Ph), 3.78 (s, 1 H, OH), 4.44 (t, J = 5.4 Hz, 1 H, Th-$CH_2$-C*H* (OC*H*$_3$)$_2$), 6.83 (d, J = 5.4 Hz, 1 H, 4-*H*-Th), 6.99 (d, J = 5.4 Hz, 1 H, 5-*H*-Th) 7.10 - 7.21 (m, 5 H, Ph-*H*).

**Eample 3: 1-Benzyl-6'-methoxy-6',7'-dihydrospiro(piperidin-4,4'-thieno[3.2-c]pyran]**

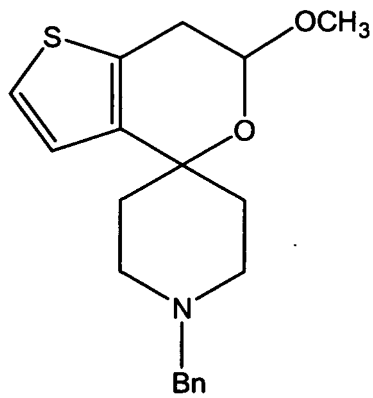

Experimental procedure

Example E (287 mg, 0.8 mmol) was dissolved in MeOH (15 mL) and mixed with trimethylorthoformiate (2 mL). After addition of p-toluene sulphonic acid (183 mg, 0.96 mmol) it was stirred for 24h under $N_2$ 24 h at RT. For purification it was alkalised with 2M NaOH and $H_2O$ (10 mL) added. Subsequently it was extracted three times with $CH_2Cl_2$ (5 mL). The organic phases were pooled and dried over $Na_2SO_4$, then it was filtered and the solvent removed under vacuum. The crude product (approx. 235 mg) was purified using flash-chromatography (2 cm, cyclohexane:ethylacetate 7:3, 15 mL, $R_f$ = 0.21).

Colourless crystalline solid, Mp. 78 ˚C, yield 199 mg (75%).

$C_{19}H_{23}NO_2S$ (329.5)

HPLC:

Method 1: 95.0 %, $R_t$ = 18.0 min
Method 2:

MS (EI): m/z = 329 ($M^+$), 298 [$M^+$-$OCH_3$], 238 [$M^+$ -$CH_2Ph$], 91 [$^+CH_2Ph$].

IR (Film): ν ($cm^{-1}$) = 3099 (C-H), 2810 (C-H), 1057 (C-O), 746 (C-H).

$^1$H-NMR ($CDCl_3$):

δ (ppm) = 1.76 (dd, J = 13.5/2.1 Hz, 1 H, $N(CH_2CH_2)_2$), 1.80 - 1.93 (m, 2 H, $N(CH_2CH_2)_2$), 2.04 (td, J = 13.4/4.5 Hz, 1 H, $N(CH_2CH_2)_2$), 2.37 (td, J = 11.6/3.7 Hz, 1 H, $N(CH_2CH_2)_2$), 2.45 (td, J = 12.4/2.6 Hz, 1 H, $N(CH_2CH_2)_2$), 2.65 - 2.73 (m, 2 H, $N(CH_2CH_2)_2$), 2.77 (dd, J = 15.6/7.5 Hz, 1 H, Th-$CH_2CHOCH_3$), 2.92 (dd, J = 15.6/3.3 Hz, 1 H, Th-$CH_2CHOCH_3$), 3.48 (d, J = 13.0 Hz, 1 H, N-$CH_2$-Ph), 3.49 (s, 3 H, Th-$CH_2CHOCH_3$), 3.54 (d, J = 13.0 Hz, 1 H, N-$CH_2$-Ph), 4.81 (dd, J = 7.2/3.3 Hz, 1 H, Th-$CH_2CHOCH_3$), 6.73 (d, J = 5.4 Hz, 1 H, 4-H-Th), 7.02 (d, J = 5.4 Hz, 1 H, 5-*H*-Th), 7.15 - 7.31 (m, 5 H, Ph-H).

**Example 2: 6'-Methoxy-6',7'-dihydrospiro[piperidin-4,4'-thieno[3,2-c]pyran (different approach)**

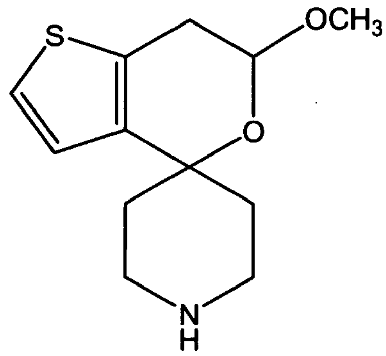

Experimental procedure

Example 3 (100 mg, 0.31 mmol) was dissolved in THF (8 mL) and cooled to -78 ˚C unter $N_2$. After addition of chloro-formic-acid-chloroethylester (45 μL, 0.40 mmol) it was stirred for 20 min, then heated to RT and THF removed under vacuum. Following that the product was mixed with MeOH (8 mL) and heated for 40 min under reflux. Subsequently the methanol was removed under vacuum. The crude product (approx. 75 mg) was purified using flash-chromatography (0.7 cm, ethyl-acetate:MeOH:$NH_3$ 90:10:2, 3 mL, $R_f$ = 0.18).

Colourless solid , Mp. 103 ˚C, yield 48 mg (65 %).

Example F: 2-{3-[4-Hydroxy-1-(2-phenylethyl)piperidin-4-yl]thiophen-2 yl}acetaldehyddimethylacetal

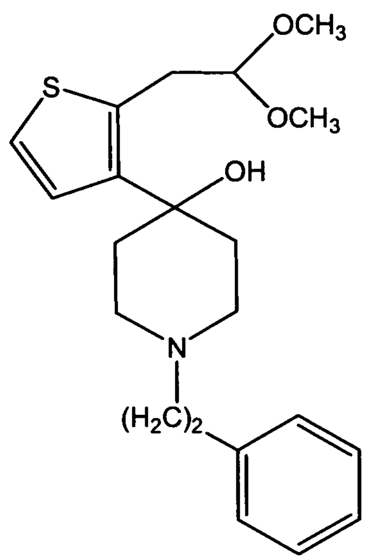

Experimental procedure

Example C (0.5 g, 2 mmol) was dissolved in THF (25 mL) and cooled to -80 ˚C under $N_2$. A solution of 1.35M n-butyllithium in hexane (1.7 mL, 2.4 mmol) was added dropwise within 2-3 min while stirring, until after 15 min 1-(2-Phenylethyl)piperidin-4-one (450 mg, 2.2 mmol) was added. After 1.5 h of stirring at -80 ˚C a further 1.5 h of stirring at RT followed. The reaction was stopped by addition of $H_2O$ (10 mL). Following that a solution of $NaHSO_3$ (10 mL, 10%) was added and extracted three times with $CH_2Cl_2$ (5 mL). The organic phases were pooled and dried over $Na_2SO_4$. Subsequently it was filtered and the solvent removed under vacuum. The crude product (approx. 1 g) was purified using flash-chromatography (3 cm, cyclohexane:ethylacetate 7:3, 30 mL, $R_f$= 0.18).

Yellowish oil, yield 62 mg (8 %).

$C_{21}H_{29}NO_3S$ (375.5)

MS (EI): m/z = 375 [$M^+$], 344 [$M^+$-$OCH_3$], 284 [$M^+$ -$CH_2$Ph], 253 [$M^+$ -$OCH_3$, -$CH_2$Ph], 91 [$^+CH_2$Ph].

IR (Film): ν ($cm^{-1}$) = 3444 (O-H); 2922, 2828 (C-H); 1070, 1055 (C-O).

$^1$H-NMR ($CDCl_3$):

δ (ppm) = 1.69 - 1.81 (m, 4 H (N($CH_2CH_2$)$_2$) (N-$CH_2CH_2$Ph)), 2.10 (td, J = 13.3/3.4 Hz, 2H, N($CH_2CH_2$)$_2$), 2.46 - 2.67 (m, 2 H, N($CH_2CH_2$)$_2$), 2.75 - 2.86 (m, 4 H (N-$CH_2CH_2$Ph) (N($CH_2CH_2$)$_2$)), 3.32 (s, 6 H, Th-$CH_2$CH($OCH_3$)$_2$), 3.39 (d, J = 5.4 Hz, 2 H, Th-$CH_2$CH($OCH_3$)$_2$), 3.90 (s, 1 H, -OH), 4.45 (t, J = 5.4 Hz, 1 H, Th-$CH_2$CH($OCH_3$)$_2$), 6.85 (d, J = 5.4 Hz, 1 H, 4-H-Th), 7.01 (d, J = 5.4 Hz, 1 H, 5-H-Th), 7.13 - 7.25 (m, 5 H, Ph-H).

**Example 4: 6'-Methoxy-1-(2-phenylethyl)-6',7'-dihydrospirorpiperidin-4,4'-thieno [3.2-c]pyran]**

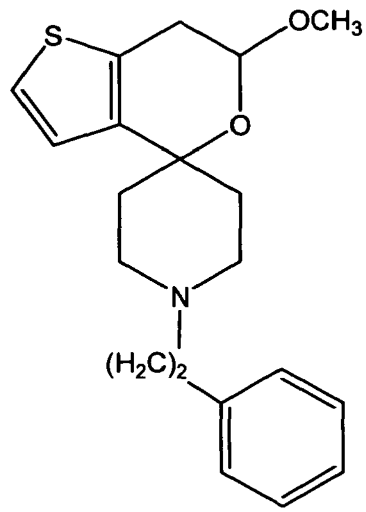

**7c**

Experimental procedure

Example F (55 mg, 0.14 mmol) was dissolved in MeOH (5 mL) and after addition of trimethylorthoformiate (1 mL) mixed with p-toluene sulphonic acid (33 mg, 1.75 mmol). After stirring for 24 h at RT it was alkalised with 2M NaOH (2 mL) and extracted three times with $CH_2Cl_2$ (5 mL). The pooled organic phases were dried over $Na_2SO_4$, then filtered and the solvent removed under vacuum. The crude product (approx. 50 mg) was purified using flash-chromatography (1 cm, cyclohexane:ethylacetate 7:3, 5 mL, $R_f$ = 0.10).

Beige crystalline solid, mp. 94 °C, yield 21 mg (44 %).

$C_{20}H_{25}NO_2S$ (343.5)

HPLC:

Method 1: 97.9 %, $R_t$ = 18.9 min
Method 2:

MS (EI): m/z = 343 [$M^+$], 312 [$M^+$-$OCH_3$), 252 [$M^+$ -$CH_2$Ph], 91 [$^+CH_2$Ph].

IR (Film): ν ($cm^{-1}$) = 3108 (C-H), 2954 (C-H), 1060 (C-O), 747 (C-H).

$^1$H-NMR ($CDCl_3$):

δ (ppm) = 1.78 - 1.98 (m, 3 H, N($CH_2CH_2$)$_2$), 2.08 (td, J = 12.6/4.4 Hz, 1 H, N($CH_2CH_2$)$_2$), 2.38 - 2.56 (m, 2 H, N ($CH_2CH_2$)$_2$), 2.57 - 2.63 (m, 2 H, N- $CH_2CH_2$-Ph), 2.76 - 2.89 (m, 5 H (Th-$CH_2$CHO$CH_3$) (N-$CH_2CH_2$-Ph) (N ($CH_2CH_2$)$_2$)), 2.94 (dd, J = 15.6/3.3 Hz, 1 H, Th-$CH_2$CHO$CH_3$), 3.51 (s, 3 H, Th-$CH_2$CHO$CH_3$), 4.82 (dd, J = 7.2/3.3 Hz, 1 H, Th-$CH_2$CHO$CH_3$), 6.75 (d, J = 5.4 Hz, 1 H, 4-H-Th), 7.04 (d, J = 5.4 Hz, 1 H, 5-H-Th), 7.15 - 7.28 (m, 5 H, Ph-H).

**Example G: 2-{3-[1-(Cyclohexylmethyl)-4-hydroxypiperidin-4-yl]thiophen-2-yl} acetaldehyddimethylacetal**

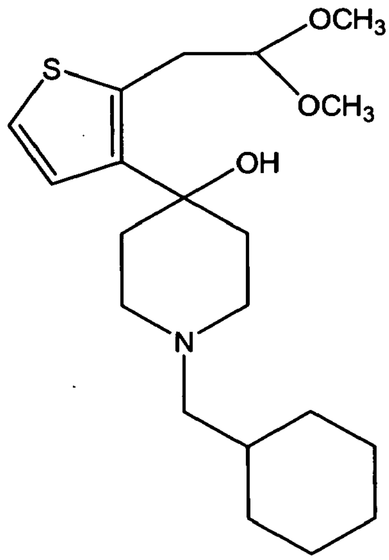

Experimental procedure

Example C (1.18 g, 4.7 mmol) was dissolved in THF (50 mL) and cooled to -78 ˚C under $N_2$. A solution of 1,5M n-butyllithium in hexane (5.2 mL, 7.0 mmol) was added dropwise within 2-3 min while stirring, until after 15 min 1-(Cyclohexylmethyl)piperidin-4-one (1.0 g, 5.2 mmol) was added. After stirring for 1.5 h at -78 ˚C it was stirred a further 2 h at RT. The reaction was stopped by addition of $H_2O$ (50 mL) beendet. Following that it was extracted three times with $CH_2Cl_2$ (10 mL). The organic phases were pooled and dried over Na2SO4, then filtered and the solvent removed under vacuum. The crude product (approx. 1.8 g) was purified using flash-chromatography (4 cm, Cyclohexane:ethylacetate 1:1, 30 mL, $R_f$= 0.21).

Colourless oil, yield 0.8 g (46 %).

$C_{20}H_{33}NO_3S$ (367.5)

MS (EI): m/z = 367 [$M^+$], 336 [$M^+$ -$OCH_3$ ], 284 [$M^+$ -cHex].

IR (Film): v ($cm^{-1}$) = 3457 (O-H), 2918 (C-H), 1053 (C-O), 719 (C-H).

$^1$H-NMR ($CDCl_3$):

δ (ppm) = 0.77 - 0.87 (m, 2 H, cHex-*H*), 1.07 - 1.20 (m, 3 H, cHex-*H*), 1.40 - 1.48 (m, 1 H, cHex-*H*), 1.55 - 1.75 (m, 5 H, cHex-*H*), 1.66 (dd, J = 13.7/2.6 Hz, 2 H, $N(CH_2CH_2)_2$), 2.03 (td, J = 12.6/3.3 Hz, 2 H, $N(CH_2CH_2)_2$), 2.11 (d, J = 7.4 Hz, 2 H, N-$CH_2$-cHex), 2.31 (t, J = 11.7 Hz, 2 H, $N(CH_2CH_2)_2$), 2.59 - 2.65 (m, 2 H, $N(CH_2CH_2)_2$), 3.29 (s, 6 H, Th-$CH_2CH(OCH_3)_2$), 3.87 (d, J = 4.2 Hz, 2 H, Th-$CH_2CH(OCH_3)_2$), 3.69 (s, 1 H, OH), 4.44 (t, J = 4.2 Hz, 1 H, Th-$CH_2CH(OCH_3)_2$), 6.83 (d, J = 5.2 Hz, 1 H, 4-H-Th), 7.00 (d, J = 5.2 Hz, 1 H, 5-H-Th).

**Example 5: 1-(Cyclohexvlmethyl)-6'-Methoxy -6',7'-dihydrospiro[piperidin-4,4'-thieno[3.2-c]pyran]**

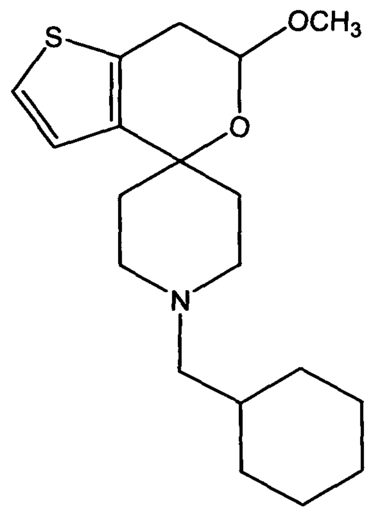

Experimental procedure

Example G (0.8 g, 2.2 mmol) was dissolved in MeOH (30 mL) and mixed with trimethyl orthoformiate (3 mL). Folowing addition of p-toluene sulphonic acid (0.5 g, 2.6 mmol) it was stirred for 3 h at RT under $N_2$. For purification it was alkalised with 2M NaOH and $H_2O$ (10 mL) added. Subsequently it was extracted three times with $CH_2Cl_2$ (10 mL) extracted. The organic phases were pooled and dried over $Na_2SO_4$, then filtered and the solvent removed under vacuum. The crude product was purified using flash-chromatography (2.5 cm, Cyclohexane:ethylacetate 7:3, 15 mL, $R_f$ = 0.45).
Colourless crystalline solid, Mp. 78 ˚C, yield 490 mg (68 %).

**Example H: 2-{3-[4-Hydroxy-1-(3-methylbutyl)piperidin-4-yl]thiophen-2-yl} acetaldehyddimethylacetal**

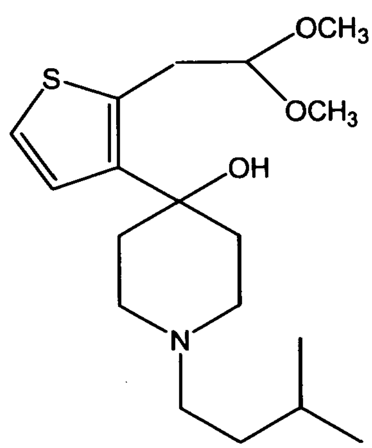

Experimental procedure

Example C (1.6 g, 6.4 mmol) was dissolved in THF (80 mL) and cooled to -78 ˚C under $N_2$. A solution of 1,5M n-butyllithium in hexane (5.15 mL, 7.7 mmol) was added dropwise within 2-3 min while stirring, until after 15 min 1-(3-Methylbutyl)piperidin-4-one (1.2 g, 7.0 mmol) was added. After 2 h of stirring at -78 ˚C another 2 h of stirring at RT was added. The reaction was stopped by addition of $H_2O$ (50 mL). Subsequently it was extracted three times with $CH_2Cl_2$ (10 mL) extracted. The organic phases were pooled and dried over Na2SO4, then filtered and the solvent removed under vacuum. The crude product (approx. 2.2 g) was purified using flash-chromatography (4 cm, Cyclohexane:ethylacetate 1:1, 50 mL, $R_f$= 0.22).

Colourless oil, yield 1.2 g (approx. 55 %, slight impurities of 1-(3-Methylbutyl)piperidin-4-one).

$C_{18}H_{31}NO_3S$ (341.5)

MS (EI): m/z = 341 [$M^+$], 310 [$M^+$-$OCH_3$], 284 [$M^+$ -$CH_2CH(CH_3)_2$].

IR (Film): ν ($cm^{-1}$) = 3446 (O-H), 2951 (C-H), 1054 (C-O), 719 (C-H).

$^1$H-NMR ($CDCl_3$):

δ (ppm) = 0.91 (d, J = 4.8 Hz, 6 H, $CH_2CH_2CH(CH_3)_2$), 1.41 - 1.45 (m, 1 H, $CH_2CH_2CH(CH_3)_2$), 1.52 - 1.62 (m, 2 H, $CH_2CH_2CH(CH_3)_2$), 1.76 (dd, J = 13.7/2.7 Hz, 2 H, $N(CH_2CH_2)_2$), 2.13 (td, J = 12.6/4.2 Hz, 2 H, $N(CH_2CH_2)_2$), 2.38 - 2.48 (m, 4 H ($CH_2CH_2CH(CH_3)_2$) ($N(CH_2CH_2)_2$)), 2.77 - 2.82 (m, 2 H, $N(CH_2CH_2)_2$), 3.37 (s, 6 H, Th-$CH_2CH(OCH_3)_2$), 3.45 (d, J = 4.2 Hz, 2 H, Th-$CH_2CH(OCH_3)_2$), 3.85 (s, 1 H, OH), 4.51 (t, J = 4.2 Hz, 1 H, Th-$CH_2CH(OCH_3)_2$), 6.90 (d, J = 5.2 Hz, 1 H, 4-H-Th), 7.06 (d, J = 5.2 Hz, 1 H, 5-H-Th).

**Example 6: 6'-Methoxy-1-(3-methylbutyl)-6',7'-dihydrospirofpiperidin-4,4'-thieno[3.2-c]pyranl**

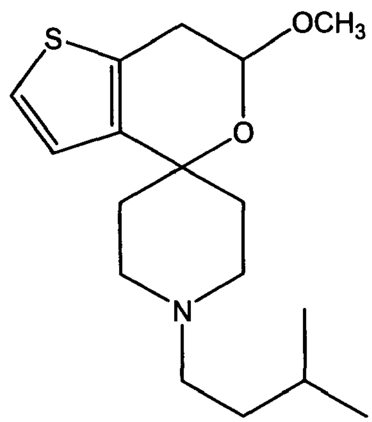

Experimental procedure

**Example H** (1.2 g, 3.5 mmol) was dissolved in MeOH (40 mL) and mixed with trimethylorthoformiate (4 mL). After addition of p-toluene sulphonic acid (0.8 g, 4.2 mmol) it was stirred under $N_2$ for 24 h at RT. For purification it was alkalised with 2M NaOH and $H_2O$ (10 mL) added. Subsequently it was extracted three times with $CH_2Cl_2$ (10 mL). The organic phases were pooled and dried over Na2SO4, then filtered and the solvent removed under vacuum. The crude product (approx. 1.1 g) was purified using flash-chromatography (4 cm, cyclohexane:ethylacetate 7:3, 30 mL, $R_f$= 0.13).

Colourless crystalline solid, Mp. 78 ˚C, yield 950 mg (88 %).

**Example 5: 1-(Cyclohexylmethyl]-6'-Methoxy-6',7'-dihydrospiro[piperidin-4,4'-thieno[3.2-c]pyran]] (alternative route)**

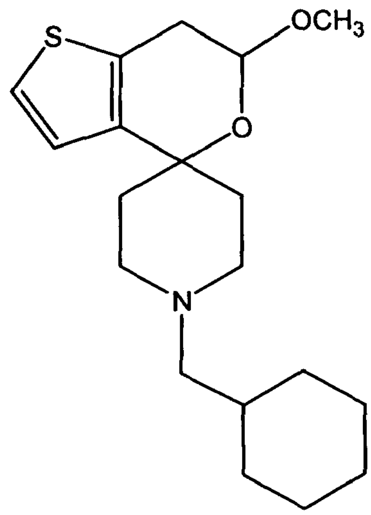

Experimental procedure

Example 2 (90 mg, 0.37 mmol) was dissolved in $CH_3CN$ (8 mL), mixed with cyclohexylmethylbromide (80 mg, 0.45 mmol) and $K_2CO_3$ (300 mg) and heated for 24 h under reflux. For purification $K_2CO_3$ was removed by a frit and the solution transferred into a separating funnel. After addition of saturated NaCl solution (10 mL) it was extracted three times with $Et_2O$ (5 mL). The organic phases were pooled and dried over Na2SO4, then filtered and the solvent removed under vacuum. The crude product (approx. 162 mg) was purified using flash-chromatography (1.5 cm, Cyclohexane: ethylacetate 7:3, 10 mL, $R_f$ = 0.45).

Colourless crystalline solid, Mp. 77 ˚C, yield 118 mg (95 %).

$C_{19}H_{29}NO_2S$ (335.5)

HPLC:

Method 1: 98.5 %, $R_t$ = 19.1 min
Method 2:

MS (EI): m/z = 335 [$M^+$], 304 [$M^+$ -$OCH_3$ ], 252 [$M^+$ - cHex].

IR (Film): ν ($cm^{-1}$) = 3102 (C-H), 2914 (C-H), 1058 (C-O), 748 (C-H).

$^1$H-NMR ($CDCl_3$):

δ (ppm) = 0.82 - 0.87 (m, 2 H, cHex-*H*), 1.12 - 1.20 (m, 2 H, cHex-*H*), 1.41 - 1.51 (m, 1 H, cHex-*H*), 1.56 - 1.86 (m, 9 H ($N(CH_2CH_2)_2$) (cHex-H), 2.05 (td, J = 13.2/4.5 Hz, 1 H, $N(CH_2CH_2)_2$), 2.12 (d, J = 7.0 Hz, 2 H, N-C$H_2$-cHex), 2.27 (td, J = 12.1/3.3 Hz, 1 H, $N(CH_2CH_2)_2$), 2.34 (td, J = 12.5/2.3 Hz, 1 H, $N(CH_2CH_2)_2$), 2.64 - 2.70 (m, 2 H, N ($CH_2CH_2)_2$), 2.78 (dd, J = 16.0/7.1 Hz, 1 H, Th-$CH_2CHOCH_3$), 2.92 (dd, J = 15.6/3.1 Hz, 1 H, Th-$CH_2CHOCH_3$), 3.51 (s, 3 H, Th-$CH_2CHOCH_3$), 4.81 (dd, J = 7.4/3.1 Hz, 1 H, Th-$CH_2CHOCH_3$), 6.72 (d, J = 5.1 Hz, 1 H, 4-H-Th), 7.02 (d, J = 5.5 Hz, 1 H, 5-H-Th).

**Example 6: 6'-Methoxy-1-(3-methylbutyl)-6',7'-dihvdrospiro[piperidin-4,4'-thieno[3.2-c]pyranl (alternative route)**

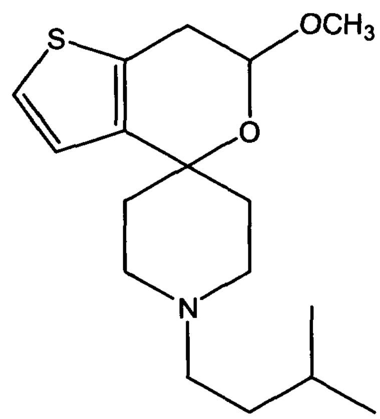

Experimental procedure

Example 2 (58 mg, 0.24 mmol) was dissolved in $CH_2Cl_2$ (5 mL), mixed with Isovaleraldehyde (22 mg, 0.25 mmol) and $NaBH(OAc)_3$ (80 mg, 0.38 mmol) and stirred for 2 h at RT. Following the end of the reaction a saturated solution of $NaHCO_3$ (5 mL) was added and extracted three times with $CH_2Cl_2$ (5 mL). The organic phases were pooled and dried over Na2SO4, then filtered and the solvent removed under vacuum. The crude product (approx. 100 mg) was purified using flash-chromatography (1 cm, cyclohexane:ethylacetate 7:3, 5 mL, $R_f$= 0.13).
Colourless crystalline solid, Mp. 38 ˚C, yield 61.5 mg (82.5 %).
$C_{17}H_{27}NO_2S$ (309.5)
HPLC:

Method 1: 98.3 %, $R_t$ = 18.1 min
Method 2:

MS (EI): m/z = 309 [$M^+$], 278 [$M^+$-$OCH_3$], 252 [$M^+$ -$C_4H_9$].

IR (Film): ν ($cm^{-1}$) = 3098 (C-H), 2949 (C-H), 1058 (C-O), 745 (C-H).
$^1$H-NMR ($CDCl_3$):

δ (ppm) = 0.85 (d, J = 6.7 Hz, 6 H, N-$CH_2CH_2CH(CH_3)_2$), 1.36 - 1.41 (m, 1 H, N-$CH_2CH_2CH(CH_3)_2$), 1.51 - 1.58 (m, 2 H, N-$CH_2CH_2CH(CH_3)_2$), 1.78 (dd, J = 13.7/2.7 Hz, 1 H, N($CH_2C\mathit{H}_2)_2$), 1.84 (td, J = 12.6/4.3 Hz, 1 H, N ($CH_2C\mathit{H}_2)_2$), 1.92 (dd, J = 14.2/2.8 Hz, 1 H, N($CH_2C\mathit{H}_2)_2$), 2.05 (td, J = 13.2/4.5 Hz, 1 H, N($CH_2C\mathit{H}_2)_2$), 2.27 - 2.42 (m, 4 H (N-$C\mathit{H}_2CH_2CH(CH_3)_2$) (N($C\mathit{H}_2CH_2)_2$)), 2.74 - 2.81 (m, 3 H (N($CH_2CH_2)_2$) (Th-$CH_2CHOCH_3$), 2.91 (dd, J = 15.6/3.3 Hz, 1 H, Th-$C\mathit{H}_2CHOCH_3$), 3.51 (s, 3 H, Th-$CH_2CH(OC\mathit{H}_3)$, 4.82 (dd, J = 7.1/3.3 Hz, 1 H, Th-$CH_2C\mathit{H}OCH_3$), 6.74 (d, J = 5.1 Hz, 1 H, 4-*H*-Th), 7.03 (d, J = 5.1 Hz, 1 H, 5-*H*-Th).

**Example 7: 6'-Methoxy-1-(3-methylbut-2-enyl)-6',7'-dihydrospiro[piperidin-4,4'-thieno[3.2-c]pyran]**

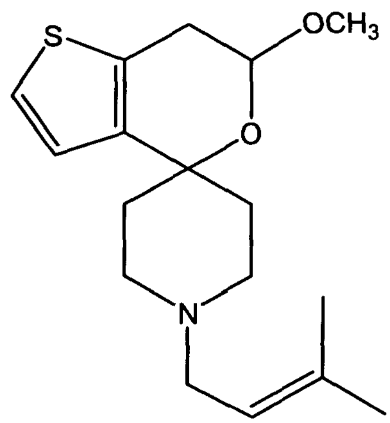

Experimental procedure

Example 2 (59 mg, 0.24 mmol) was dissolved in $CH_3CN$ (5 mL), mixed with 1-Bromo-3-methyl-2-butene (45 mg, 0.3 mmol) and $K_2CO_3$ (200 mg) and heated for 24 h under reflux. For purification $K_2CO_3$ was removed by a frit. After addition of saturated NaCl-solution (5 mL) it was extracted three times with $Et_2O$ (5 mL). The organic phases were pooled and dried over Na2SO4, then filtered and the solvent removed under vacuum. The crude product (approx. 100 mg) was purified using flash-chromatography (1 cm, cyclohexane:ethylacetate 7:3, 5 mL, $R_f$ = 0.15).

Colourless crystalline solid, Mp. 40 ˚C, yield: 16 mg (21 %).

$C_{17}H_{25}NO_2S$ (307.5)

HPLC:

Method 1: 97.5 %, $R_t$ = 17.8 min

Method 2:

MS (EI): m/z = 307 [$M^+$], 276 [$M^+$-$OCH_3$], 239 [$M^+$ -$CH_2CH=C(CH_3)_2$].

IR (Film): v ($cm^{-1}$) = 3096 (C-H), 2813 (C-H), 1675 (C=C), 1058 (C-O), 744 (C-H).

$^1$H-NMR ($CDCl_3$):

δ (ppm) = 1.60 (s, 3 H, N-$CH_2CH=C(C\mathit{H}_3)_2$), 1.68 (s, 3 H, N-$CH_2CH=C(C\mathit{H}_3)_2$), 1.79 (dd, J = 13.7/2.7 Hz, 1 H, N($CH_2C\mathit{H}_2)_2$), 1.84 (td, J = 12.5/4.5 Hz, 1H, N($CH_2C\mathit{H}_2)_2$), 1.91 (dd, J = 13.7/2.5 Hz, 1 H, N($CH_2C\mathit{H}_2)_2$), 2.05 (td, J = 13.2/4.4 Hz, 1 H, N($CH_2C\mathit{H}_2)_2$), 2.32 (td, J = 12.0/3.3 Hz, 1 H, N($C\mathit{H}_2CH_2)_2$), 2.41 (td, J = 12.6/2.6 Hz, 1 H, N($C\mathit{H}_2CH_2)_2$), 2.76 - 2.81 (m, 3 H (N($C\mathit{H}_2CH_2)_2$) (Th-$C\mathit{H}_2CHOCH_3$), 2.90 - 2.97 (m, 3 H (Th-$C\mathit{H}_2CHOCH_3$) (N-$C\mathit{H}_2CH=C(CH_3)_2$)), 3.52 (s, 3 H, Th-$CH_2CHOC\mathit{H}_3$), 4.82 (dd, J = 7.1/3.3 Hz, 1 H, Th-$CH_2C\mathit{H}OCH_3$), 5.25 (t, J = 7.8 Hz, 1 H, N-$CH_2C\mathit{H}=C(CH_3)_2$), 6.73 (d, J = 5.1 Hz, 1 H, 4-H-Th), 7.03 (d, J = 5.1 Hz, 1 H, 5-H-Th).

**Example 8: 1-Butyl-6'-methoxy-6',7'-dihydrospiro[piperidin-4,4'-thieno[3.2-c]pyran]**

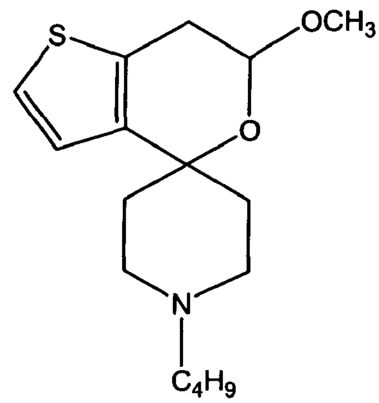

**9f**

Experimental procedure

Example 2 (90 mg, 0.37mmol) was dissolved in $CH_3CN$ (8 mL), mixed with butylbromide (79 mg, 0.57 mmol) and $K_2CO_3$ (300 mg) and heated for 24 h under reflux. For purification $K_2CO_3$ was removed over a frit, and the solution transferred to separating funnel. After addition of a saturated NaCl-solution (10 mL) it was extracted three times with $Et_2O$ (5 mL). The organic phases were pooled and dried over $Na_2SO_4$, then filtered and the solvent removed under vacuum. The crude product (approx. 110 mg) was purified using flash-chromatography (1 cm, Cyclohexane:ethylacetate 7:3, 10 mL, $R_f$ = 0.14).

Colourless crystalline solid, Mp. 47°C, yield 70 mg (65 %).

$C_{16}H_{25}NO_2S$ (295.4)

HPLC:

Method 1: 97.4 %, $R_t$ = 16.9 min
Method 2:

MS (EI): m/z = 295 [$M^+$], 264 [$M^+$-$OCH_3$], 252 [$M^+$ -$C_3H_7$].

IR (Film): v ($cm^{-1}$) = 2926 (C-H); 2807 (N-*C*-*H*), 1060 (C-O), 719 (C-H).

$^1$H-NMR ($CDCl_3$):

δ (ppm) = 0.94 (t, J = 7.2 Hz, 3H, N-$CH_2CH_2CH_2CH_3$), 1.36 (sext, J = 7.2 Hz, 2H, N-$CH_2CH_2CH_2CH_3$), 1.54 (quin, J = 7.2 Hz, 2H, N-$CH_2CH_2CH_2CH_3$), 1.85 (dd, J = 13.7/2.6 Hz, 1 H, N($CH_2CH_2$)$_2$), 1.93 (td, J = 14.2/4.4 Hz, 1 H, N($CH_2CH_2$)$_2$), 1.99 (dd, J = 13.7/2.6 Hz, 1 H, N($CH_2CH_2$)$_2$), 2.14 (td, J = 13.2/4.5 Hz, 1 H, N($CH_2CH_2$)$_2$), 2.37 - 2.45 (m, 3 H (N($CH_2CH_2$)$_2$) ($CH_2CH_2CH_2CH_3$)), 2.49 (td, J = 12.4/2.6 Hz, 1 H, N($CH_2CH_2$)$_2$), 2.80 - 2.89 (m, 3 H (N($CH_2CH_2$)$_2$) (Th-$CH_2CHOCH_3$)), 2.99 (dd, J = 15.6/3.3 Hz, 1 H, Th-$CH_2CHOCH_3$), 3.57 (s, 3 H, Th-$CH_2CHOCH_3$), 4.88 (dd, J = 7.8/3.3 Hz, 1 H, Th-$CH_2CHOCH_3$), 6.80 (d, J = 5.1 Hz, 1 H, 4-*H*-Th), 7.08 (d, J = 5.4 Hz, 1 H, 5-*H*-Th).

**Example 9: 6'-Methoxy-1-pentyl-6',7'-dihydrospiro[piperidin-4,4'-thieno[3.2-c]pyran]**

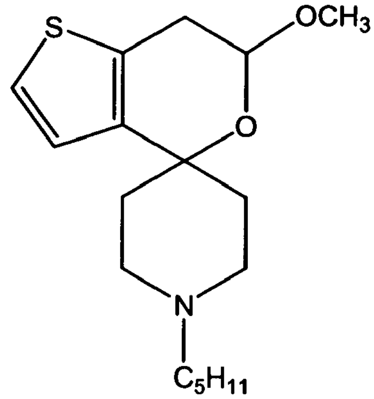

Experimental procedure

Example 2 (62 mg, 0.26 mmol) was dissolved in $CH_2Cl_2$ (5 mL), mixed with valeraldehyde (24 mg, 0.28 mmol) and $NaBH(OAc)_3$ (80 mg, 0.38 mmol) and stirred for 2 h at RT. After the finishing of the reaction saturated $NaHCO_3$-solution (5 mL) was added and three times extracted with $CH_2Cl_2$ (5 mL). The organic phases were pooled and dried over Na2SO4, then filtered and the solvent removed under vacuum. The crude product (approx. 100 mg) was purified using flash-chromatography (1 cm, cyclohexane:ethylacetate 7:3, 5 mL, $R_f$ = 0.2).

Colourless crystalline solid, Mp. 43 °C, yield 69 mg (88 %).

$C_{17}H_{27}NO_2S$ (309.5)

HPLC:

Method 1: 99.1 %, $R_t$ = 18.4 min
Method 2:

MS (EI): m/z = 309 [$M^+$], 278 [$M^+$-$OCH_3$], 252 [$M^+$ -$C_4H_9$].

IR (Film): ν ($cm^{-1}$) = 3098 (C-H), 2926 (C-H), 1058 (C-O), 744 (C-H).

$^1$H-NMR ($CDCl_3$):

δ (ppm) = 0.84 (t, J = 6.6 Hz, 3 H, N-$CH_2CH_2CH_2CH_2CH_3$), 1.23 - 1.28 (m, 4 H, N-$CH_2CH_2CH_2CH_2CH_3$), 1.42 - 1.52 (m, 2 H, N-$CH_2CH_2CH_2CH_2CH_3$), 1.78 (dd, J = 13.7/2.7 Hz, 1 H, N($CH_2CH_2$)$_2$), 1.84 (td, J = 12.6/4.3 Hz, 1 H, N($CH_2CH_2$)$_2$), 1.92 (dd, J = 14.2/2.8 Hz, 1 H, N($CH_2CH_2$)$_2$), 2.05 (td, J = 13.2/4.4 Hz, 1 H, N($CH_2CH_2$)$_2$), 2.28 - 2.35 (m, 3 H (N-$CH_2CH_2CH_2CH_2CH_3$) (N($CH_2CH_2$)$_2$)), 2.39 (td, J = 12.6/2.6 Hz, 1 H, N($CH_2CH_2$)$_2$), 2.73 - 2.81 (m, 3 H (N($CH_2CH_2$)$_2$) (Th-$CH_2CHOCH_3$), 2.92 (dd, J = 15.6/3.2 Hz, 1 H, Th-$CH_2CHOCH_3$), 3.51 (s, 3 H, Th-$CH_2CHOCH_3$), 4.82 (dd, J = 7.4/3.1 Hz, 1 H, Th-$CH_2CHOCH_3$), 6.74 (d, J = 5.1 Hz, 1 H, 4-H-Th), 7.03 (d, J = 5.5 Hz, 1 H, 5-H-Th).

**Example 10: 6'-Methoxy-1-octyl -6',7'-dihydrospiro[piperidin-4,4'-thieno[3,2-c]pyran]**

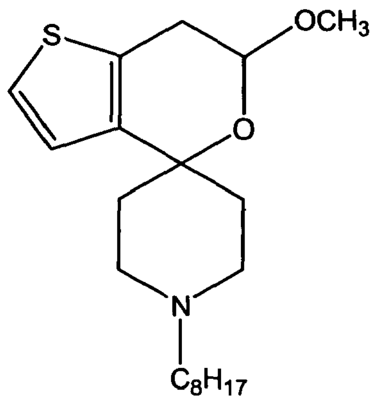

Experimental procedure

Example 2 (180 mg, 0.75 mmol) was dissolved in $CH_3CN$ (20 mL), mixed with octylbromide (174 mg, 0.9 mmol) and $K_2CO_3$ (620 mg) and heated for 24 h under reflux. For purification $K_2CO_3$ was separated over a frit and the solution transferred into a separating funnel. After addition of saturated NaCl-solution (10 mL) it was extracted three times with $Et_2O$ (5 mL). The organic phases were pooled and dried over $Na_2SO_4$, then filtered and the solvent removed under vacuum. The crude product (approx. 237 mg) was purified using flash-chromatography (2 cm, cyclohexane:ethylacetate 7:3, 10 mL, $R_f$ = 0.37).

Colourless crystalline solid, Mp. 48 ˚C, yield 229 mg (87 %).

$C_{20}H_{33}NO_2S$ (351.1)

HPLC:

Method 1: 99.1 %, $R_t$ = 21.8 min
Method 2:

MS (EI): m/z = 351 [$M^+$], 320 [$M^+$-$OCH_3$], 252 [$M^+$ -$C_7H_{15}$].

IR (Film): v ($cm^{-1}$) = 3092 (C-H), 2918 (C-H), 1058 (C-O), 742 (C-H).

$^1$H-NMR ($CDCl_3$):

δ (ppm) = 0.81 (t, J = 6.0 Hz, 3 H, N-$(CH_2)_7$-$CH_3$), 1.12 (m, 10 H, N-$CH_2CH_2(CH_2)_5CH_3$). 1.43 (m, 2H, N-$CH_2CH_2C_6H_{13}$), 1.78 (dd, J = 13.5/2.2 Hz, 1 H, N($CH_2CH_2)_2$), 1.88 (td, J = 12.2/4.2 Hz, 1 H, N($CH_2CH_2)_2$), 1.93 (dd, J = 13.5/2.2 Hz, 1 H, N($CH_2CH_2)_2$), 2.05 (td, J = 13.2/4.5 Hz, 1 H, N($CH_2CH_2)_2$), 2.25 - 2.45 (m, 4 H (N($CH_2CH_2)_2$) (N-$CH_2(CH_2)_6CH_3$)), 2.77 (dd, J = 15.3/7.2 Hz, 1 H, Th-$CH_2CHOCH_3$), 2.75 - 2.80 (m, 2 H, N($CH_2CH_2)_2$), 2.94 (dd, J = 15.3/3.3 Hz, 1 H, Th-$CH_2CHOCH_3$), 3.51 (s, 3 H, Th-$CH_2CHOCH_3$), 4.82 (dd, J = 7.2/3.3 Hz, 1 H, Th-$CH_2$-CHO$CH_3$), 6.73 (d, J = 5.4 Hz, 1 H, 4-*H*-Th), 7.02 (d, J = 5.4 Hz, 1 H, 5-*H*-Th).

**Example 11: 3'-Methoxy-1-(3-phenylpropyl)-6',7'-dihydrospiro[piperidin-4,4'-thieno[3.2-c]pyran]**

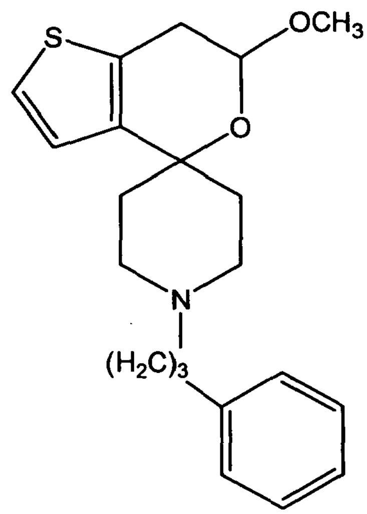

Experimental procedure

Example 2 (120 mg, 0.5 mmol) was dissolved in $CH_3CN$ (15 mL), mixed with 3-phenylpropylbromide (120 mg, 0.6 mmol) and $K_2CO_3$ (414 mg) and heated for 24 h under reflux. For purification $K_2CO_3$ was removed by a frit and the solution transferred into a separating funnel. After addition of saturated NaCl-solution (10 mL) it was extracted three times with $Et_2O$. The organic phases were pooled and dried over Na2SO4, then filtered and the solvent removed under vacuum. The crude product was purified using flash-chromatography (2 cm, cyclohexane:ethylacetate 7:3, 10 mL, $R_f$ = 0.2).

Colourless Solid, Mp. 59 ˚C, yield 68 mg (38 %).

$C_{21}H_{27}NO_2S$ (357.5)

HPLC:

Method 1: %, $R_t$ = min
Method 2:

MS (EI): m/z = 357 [$M^+$], 326 [$M^+$-$OCH_3$], 252 [$M^+$ -$(CH_2)_2$-Ph].

IR (Film): ν ($cm^{-1}$) = 3097 (C-H), 2913 (C-H), 1062 (C-O), 692 (C-H).

$^1$H-NMR ($CDCl_3$):

δ (ppm) = 1.75 - 1.89 (m, 4 H ($N(CH_2CH_2)_2$) (N-$CH_2CH_2CH_2$-Ph)), 1.92 (dd, J = 13.8/2.8 Hz, 1 H, $N(CH_2CH_2)_2$), 2.04 (td, J = 13.8/4.2 Hz, 1 H, $N(CH_2CH_2)_2$), 2.26 - 2.46 (m, 4 H ($N(CH_2CH_2)_2$) (N-$CH_2CH_2CH_2$-Ph)), 2.59 (t, J = 7.8 Hz, 2 H, N-$CH_2CH_2CH_2$-Ph), 2.71 - 2.79 (m, 2 H, $N(CH_2CH_2)_2$), 2.77 (dd, J = 15.6/7.2 Hz, 1 H, Th-$CH_2CHOCH_3$), 2.92 (dd, J = 15.6/3.3 Hz, 1 H, Th-$CH_2CHOCH_3$), 3.50 (s, 3 H, Th-$CH_2CHOCH_3$), 4.81 (dd, J = 7.2/3.3 Hz, 1 H, Th-$CH_2CHOCH_3$), 6.73 (d, J = 5.1 Hz, 1 H, 4-*H*-Th), 7.02 (d, J = 5.4 Hz, 1 H, 5-*H*-Th), 7.10 - 7.25 (m, 5 *H*, Ph-*H*).

**Example 12: 3'-Methoxy-1-(4-phenylbutyl)-6',7'-dihydrospiro[piperidin-4,4' thieno[3.2-c]pyran]**

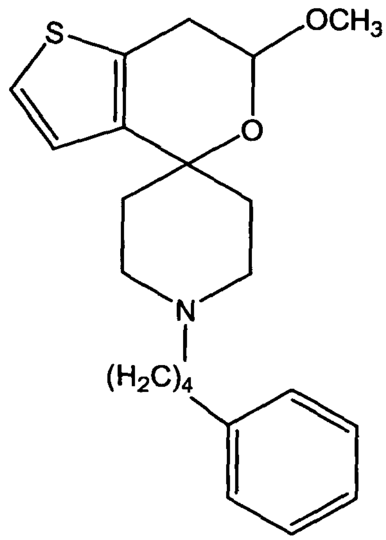

Experimental procedure

Example 2 (89 mg, 0.36 mmol) was dissolved in $CH_3CN$ (10 mL), mixed with 4-phenylbutylchloride (76 mg, 0.45 mmol) and $K_2CO_3$ (310 mg) and heated for 24 h under reflux. For purification $K_2CO_3$ was removed by a frit and the solution transferred into a separating funnel. After addition of saturated NaCl-solution (10 mL) it was extracted three times with $Et_2O$. The organic phases were pooled and dried over $Na_2SO_4$, then filtered and the solvent removed under vacuum. The crude product (approx. 195 mg) was purified using flash-chromatography (1.5 cm, Cyclohexane:ethylacetate 7:3, 5 mL, $R_f$ = 0.13).

Colourless crystalline solid, Mp. 75°C, yield 46 mg (34 %).

$C_{22}H_{29}NO_2S$ (371.5)

HPLC:

Method 1: %, $R_t$ = min
Method 2:

MS (EI): m/z = 371 [$M^+$], 340 [$M^+$-$OCH_3$ ], 252 [$M^+$ -$(CH_2)_3$Ph].

IR (Film): v ($cm^{-1}$) = 3096 (C-H), 2922 (C-H), 1059 (C-O), 745 (C-H).

$^1$H-NMR ($CDCl_3$):

δ (ppm) = 1.49 - 1.63 (m, 4 H, N-$CH_2CH_2CH_2CH_2$-Ph), 1.78 (dd, J = 13.7/2.7 Hz, 1 H, N($CH_2CH_2$)$_2$), 1.85 (td, J = 12.4/4.5 Hz, 1 H, N($CH_2CH_2$)$_2$), 1.91 (dd, J = 13.7/2.7 Hz, 1 H, N($CH_2CH_2$)$_2$), 2.04 (td, J = 13.3/4.3 Hz, 1 H, N ($CH_2CH_2$)$_2$), 2.26 - 2.45 (m, 4 H (N($CH_2CH_2$)$_2$) (N-CH2CH2CH2C$H_2$-Ph)), 2.58 (t, J = 7.2 Hz, 2 H, N-$CH_2(CH_2)_3$-Ph), 2.69 - 2.81 (m, 3 H (N($CH_2CH_2$)$_2$) (Th-$CH_2$CHOCH$_3$), 2.92 (dd, J = 15.6/3.3 Hz, 1 H, Th-$CH_2$CHOCH$_3$), 3.51 (s, 3 H, Th-$CH_2$CHOC$H_3$), 4.81 (dd, J = 7.2/3.3 Hz, 1 H, Th-$CH_2$C*H*OCH$_3$), 6.72 (d, J = 5.4 Hz, 1 H, 4-*H*-Th), 7.02 (d, J = 5.4 Hz, 1 H, 5-*H*-Th), 7.10 - 7.23 (m, 5 H, Ph-*H*).

**Example 13: 1-(4-Flourbenzyl)-6'-Methoxy -6',7'-dihydrospiro[piperidin-4,4'-thieno[3.2-c]pyran]**

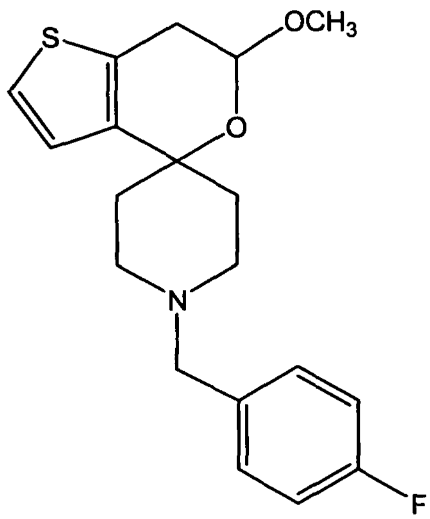

Experimental procedure

Example 2 (129 mg, 0.5 mmol) was dissolved in $CH_3CN$ (20 mL), mixed with 4-fluorobenzylchloride (87 mg, 0.6 mmol) and $K_2CO_3$ (414 mg) and heated for 24 h under reflux. For purification $K_2CO_3$ was removed by a frit and the solution transferred into a separating funnel. After addition of saturated NaCl-solution (10 mL) it was extracted three times with Et20. The organic phases were pooled and dried over Na2SO4, then filtered and the solvent removed under vacuum. The crude product was purified using flash-chromatography (2 cm, cyclohexane:ethylacetate 7:3, 10 mL, $R_f$ = 0.32).

Colourless crystalline solid, Mp. 83 °C, yield 83 mg (48 %).

$C_{19}H_{22}FNO_2S$ (347.4)

HPLC:

Method 1: 98.8 %, $R_t$ = 18.6 min
Method 2:

MS (EI): m/z = 347 [$M^+$], 316 [$M^+$ -$OCH_3$], 238 [$M^+$ -$CH_2Ph$-$_p$F].

IR (Film): v ($cm^{-1}$) = 3099 (C-H), 2812 (C-H), 1057 (C-O), 746 (C-H).

$^1$H-NMR ($CDCl_3$):

δ (ppm) = 1.76 (dd, J = 13.6/2.7 Hz, 1 H, N($CH_2CH_2$)$_2$), 1.83 (td, J = 12.2/4.0 Hz, 1 H, N($CH_2CH_2$)$_2$), 1.89 (dd, J = 13.6/2.5 Hz, 1 H, N($CH_2CH_2$)$_2$), 2.05 (td, J = 13.2/4.5 Hz, 1 H, N($CH_2CH_2$)$_2$), 2.35 (td, J = 12.6/3.3 Hz, 1 H, N ($CH_2CH_2$)$_2$), 2.43 (td, J = 12.6/2.6 Hz, 1 H, N($CH_2CH_2$)$_2$), 2.62 - 2.72 (m, 2 H, N($CH_2CH_2$)$_2$), 2.79 (dd, J = 15.6/7.2 Hz, 1 H, Th-$CH_2CHOCH_3$), 2.92 (dd, J = 15.6/3.3 Hz, 1 H, Th-$CH_2CHOCH_3$), 3.44 (d, J = 13.3 Hz, 1 H, N-$CH_2$Ph$_{(4-F)}$), 3.47 (d, J = 13.3 Hz, 1 H, N-$CH_2$Ph$_{(4-F)}$), 3.49 (s, 3 H, $OCH_3$), 4.80 (dd, J = 7.2/3.3 Hz, 1 H, Th-$CH_2CHOCH_3$), 6.72 (d, J = 5.1 Hz, 1 H, 4-*H*-Th), 6.91 - 6-98 (m, 2 H, $_{(4-F)}$Ph-*H*), 7.02 (d, J = 5.1 Hz, 1 H, 5-*H*-Th), 7.23 - 7.29 (m, 2 H, $_{(4-F)}$Ph-*H*).

**Example 14: 6'-Methoxy-1-(4-methoxybenzyl)-6',7'-dihydrospiro[piperidin-4,4'-thieno[3.2-c]pyran]**

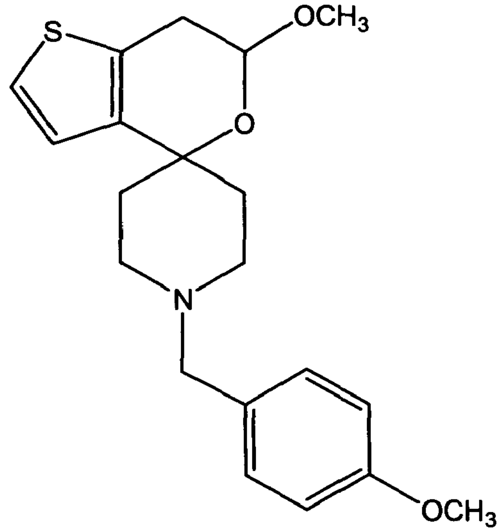

Experimental procedure

Example 2 (70 mg, 0.29 mmol) was dissolved in $CH_2Cl_2$ (5 mL), mixed with anisaldehyde (45 mg, 0.33 mmol) and $NaBH(OAc)_3$ (75 mg, 0.35 mmol) and stirred for 4 h at RT. After the end of the reaction a solution of $NaHCO_3$ (5 mL) was added and extracted three times with $CH_2Cl_2$ (5 mL). The organic phases were pooled and dried over Na2SO4, then filtered and the solvent removed under vacuum. The crude product (approx. 136 mg) was purified using flash-chromatography (1 cm, cyclohexane:ethylacetate 7:3, 5 mL, $R_f$ = 0.16).

Colourless crystalline solid, Mp. 91 ˚C, yield 94 mg (91 %).

$C_{20}H_{25}NO_3S$ (359.5)

HPLC:

Method 1: 96.8 %, Rt = 18.6 min

Method 2:

MS (EI): m/z = 359 [$M^+$], 297 [$M^+$ -$OCH_3$ (2x)], 238 [$M^+$ -$CH_2Ph$-$_pOCH_3$].

IR (Film): ν ($cm^{-1}$) = 3096 (C-H), 2926 (C-H), 1060 (C-O), 719 (C-H).

$^1H$-NMR ($CDCl_3$):

δ (ppm) = 1.75 (dd, J = 13.7/2.5 Hz, 1 H, N($CH_2CH_2$)$_2$), 1.83 (td, J = 12.5/4.5 Hz, 1 H, N($CH_2C\mathit{H}_2$)$_2$), 1.88 (dd, J = 13.7/2.5 Hz, 1 H, N($CH_2C\mathit{H}_2$)$_2$), 2.03 (td, J = 13.2/4.2 Hz, 1 H, N($CH_2C\mathit{H}_2$)$_2$), 2.34 (td, J = 12.0/3.1 Hz, 1 H, N ($C\mathit{H}_2CH_2$)$_2$), 2.43 (td, J = 12.5/2.6 Hz, 1 H, N($C\mathit{H}_2CH_2$)$_2$), 2.65 - 2.75 (m, 2 H, N($C\mathit{H}_2CH_2$)$_2$), 2.77 (dd, J = 15.6/7.1 Hz, 1 H, Th-$C\mathit{H}_2CHOCH_3$), 2.92 (dd, J = 15.6/3.3 Hz, 1 H, Th-$C\mathit{H}_2CHOCH_3$), 3.43 (d, J = 14.0 Hz, 1 H, N-$C\mathit{H}_2Ph_{(4-OMe)}$), 3.47 (d, J = 14.0 Hz, 1 H, N-$C\mathit{H}_2Ph_{(4-OMe)}$), 3.49 (s, 3 H, Th-$CH_2CHOC\mathit{H}_3$), 3.74 (s, 3 H, Ph-$OC\mathit{H}_3$), 4.80 (dd, J = 7.1/3.3 Hz, 1 H, Th-$CH_2C\mathit{H}OCH_3$), 6.72 (d, J = 5.5 Hz, 1 H, 4-*H*-Th), 6.80 (d, J = 8.4 Hz, 2 H, $_{(4-OMe)}$Ph-*H*), 7.02 (d, J = 5.1 Hz, 1 H, 5-*H*-Th), 7.20 (d, J = 8.4 Hz, 2 H, $_{(4-OMe)}$Ph-*H*).

**Example 15: 6'-Methoxy-1-(thien-2-ylmethyl)-6',7'-dihydrospiro[piperidin-4,4'-thieno[3.2-c]pyran]**

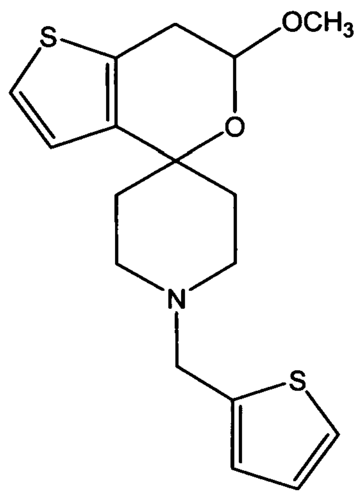

Experimental procedure

Example 2 (58 mg, 0.24 mmol) was dissolved in $CH_2Cl_2$ (5 mL), mixed with thiophene-2-carbaldehyde (31 mg, 0.28 mmol) and $NaBH(OAc)_3$ (80 mg, 0.38 mmol) and stirred for 2 h at RT. After the end of the reaction a saturated solution of $NaHCO_3$ (5 mL) was added and it was extracted three times with $CH_2Cl_2$ (5 mL). The organic phases were pooled and dried over Na2SO4, then filtered and the solvent removed under vacuum. The crude product (approx. 100 mg) was purified using flash-chromatography (1 cm, cyclohexane:ethylacetate 7:3, 5 mL, $R_f$ = 0.34).

Colourless crystalline solid, Mp. 73 ˚C, yield 69 mg (82 %).

$C_{17}H_{21}NO_2S_2$ (335.5)

HPLC:

Method 1: 98.3 %, $R_t$ = 17.5 min
Method 2:

MS (EI): m/z = 335 [$M^+$], 304 [$M^+$-$OCH_3$], 238 [$M^+$ -$CH_2$Th], 97 [$^+CH_2$Th].

IR (Film): v ($cm^{-1}$) = 3100 (C-H), 2809 (C-H), 1058 (C-O), 749 (C-H).

$^1$H-NMR ($CDCl_3$):

δ (ppm) = 1.77 (dd, J = 13.6/2.7 Hz, 1 H, $N(CH_2CH_2)_2$), 1.84 (td, J = 12.2/4.3 Hz, 1 H, $N(CH_2CH_2)_2$), 1.91 (dd, J = 14.0/2.8 Hz, 1 H, $N(CH_2CH_2)_2$), 2.05 (td, J = 13.2/4.5 Hz, 1 H, $N(CH_2CH_2)_2$), 2.42 (td, J = 11.8/3.4 Hz, 1 H, N$(CH_2CH_2)_2$), 2.51 (td, J = 12.6/2.6 Hz, 1 H, $N(CH_2CH_2)_2$), 2.73 - 2.81 (m, 3 H ($N(CH_2CH_2)_2$) ($Th_a$-$CH_2CHOCH_3$), 2.91 (dd, J = 15.6/3.1 Hz, 1 H, $Th_a$-$CH_2CHOCH_3$), 3.48 (s, 3 H, $Th_a$-$CH_2CHOCH_3$), 3.72 (d, J = 13.7 Hz, 1 H, N-$CH_2$-$Th_b$) 3.76 (d, J = 13.7 Hz, 1 H, N-$CH_2$-$Th_b$), 4.81 (dd, J = 7.8/3.1 Hz, 1 H, $Th_a$ $CH_2CHOCH_3$), 6.73 (d, J = 5.1 Hz, 1 H, 4-H-$Th_a$), 6.88 (m, 2 H (3-H-$Th_b$) (4-H-$Th_b$)), 7.02 (d, J = 5.5 Hz, 1 H, 5-H-$Th_a$), 7.17 (dd, J = 4.7/1.6 Hz, 1 H, 5-H-$Th_b$).

**Example I: 1-(Cyclohexylmethyl)piperidin-4-one**

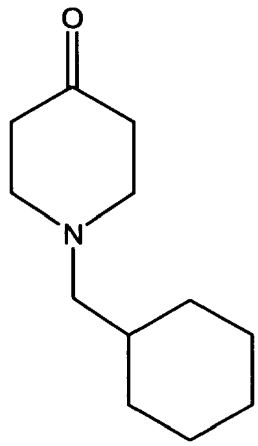

**5d**

Experimental procedure

Piperidin-4-one hydrochloride monohydrate (0.7 g, 4.55 mmol) was dissolved in $CH_3CN$:$H_2O$ 1:1 (15 mL), mixed with cyclohexylmethylbromide (0.96 mg, 5.47 mmol) and $K_2CO_3$ (3.8 g) and heated for 34 h under reflux. For purification it was extracted three times with $CH_2Cl_2$ (10 mL) and the organic phase dried over $Na_2SO_4$. Following filtration the solvent was removed under vacuum and the crude product purified by chromatography (3 cm, cyclohexane: ethylacetate 7:3, 15 mL, $R_f$ = 0.40).

Colourless oil, yield 656 mg (74 %).

$C_{12}H_{21}NO$ (195.1)

MS (EI): m/z = 195 [$M^+$], 112 [$M^+$-cHex].

IR (Film): v ($cm^{-1}$) = 2919 (C-H), 1718 (C=O), 758 (C-H).

$^1$H-NMR ($CDCl_3$):

d (ppm) = 0.78 - 0.88 (m, 2 H, cHex-*H*), 1.05 - 1.25 (m, 3 H, cHex-*H*), 1.38 - 1.43 (m, 1 H, cHex-H), 1.58 - 1.69 (m, 3 H, cHex-*H*), 1.61 - 1.68 (m, 2 H, cHex-*H*), 2.17 (d, J = 5.4 Hz, 2 H, N-C$H_2$-cHex), 2.38 (t, J = 4.5 Hz, 4 H, N($CH_2CH_2$)$_2$), 2.63 (t, J = 4.5 Hz, 4 H, N(C$H_2CH_2$)$_2$).

**1-(3-Methylbutyl)piperidin-4-on**

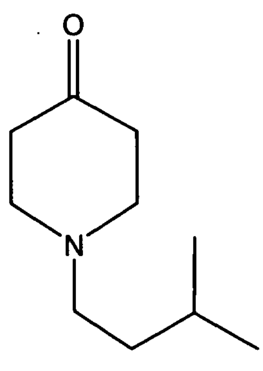

**5e**

Experimental procedure

Piperidin-4-onhydrochlorid Monohydrat (1.5 g, 9.76 mmol) wurde in $CH_3CN$:$H_2O$ 1:1 (50 mL) gelöst, mit 1-Brom-3-methylbutan (1.77 g, 11.7 mmol) und $K_2CO_3$ (8.1 g) versetzt und 18 h rückfließend erhitzt. For purification it was extracted three times with $CH_2Cl_2$ (10 mL) and the organic phases dried over $Na_2SO_4$. Following filtration the solvent was removed under vacuum and the crude product purified by flash-chromatography (3 cm, cyclohexane:ethylacetate 7:3, 30 mL, $R_f$ = 0.34).

Colourless oil, yield 1.21 g (73 %).

$C_{10}H_{19}NO$ (169.3)

MS (El): m/z = 169 [$M^+$], 112 [$M^+$ -$CH_2CH(CH_3)_2$].

IR (Film): ν ($cm^{-1}$) = 2953 (C-H), 1718 (C=O), 756 (C-H).

$^1H$-NMR ($CDCl_3$):

δ (ppm) = 0.92 (d, J = 5.1 Hz, 6 H, N-$CH_2CH_2CH(C\mathit{H}_3)_2$), 1.38 - 1.44 (m, 1 H, $CH_2CH_2C\mathit{H}(CH_3)_2$), 1.59 - 1.68 (m, 2 H, N-$CH_2C\mathit{H}_2CH(CH_3)_2$), 2.45 - 2.52 (m, 6 H ($N(CH_2C\mathit{H}_2)_2$) (N-$C\mathit{H}_2CH_2CH(CH_3)_2$)), 2.86 (t, J = 4.5 Hz, 4 H, N ($C\mathit{H}_2CH_2)_2$).

**Example 16: 1-Benzylspiro[piperidin-4,4'-thieno[3,2-c]pyranl**

**Example 17: 1-Benzyl-6',7'-dihydrospiro[piperidin-4,4'-thieno[3,2-c]pyran]-6'-carbonitrile**

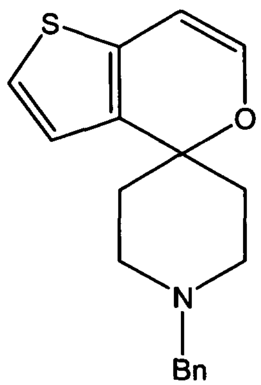

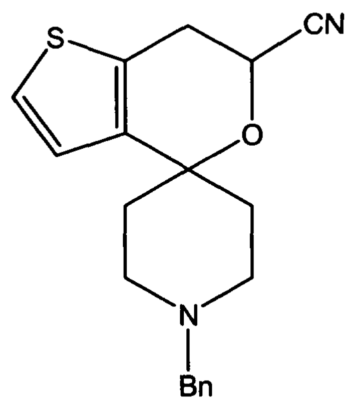

Experimental procedure

Example 3 (329 mg, 1 mmol) was dissolved in $CH_2Cl_2$ (20 mL) and cooled to -20 °C under $N_2$. Then TMS-CN (1 mL, 8 mmol) and $BF_3 \cdot Et_2O$ (0.2 mL, 1.6 mmol) were added consecutively through a septum and stirred for 30 min at -20 °C, and subsequently for 1 h at 0 °C in an ice bath. For purification MeOH (3 mL) and 2M NaOH (3 mL) were added (pH > 9) and then extracted three times with $CH_2Cl_2$ (5 mL). The organic phases were pooled and dried over Na2SO4, then filtered and the solvent removed under vacuum. The crude product was purified using flash-chromatography (2 cm, cyclohexane:ethylacetate 7:3, 5 mL, $R_f$ (**11**) = 0.42, $R_f$ (**10**) = 0.34).

**Example 16:** Colourless oil, yield 116 mg (38 %).

$C_{18}H_{19}NOS$ (297.4)

HPLC:

Method 1: 97.0 %, $R_t$ = 18.8 min
Method 2:

MS (El): m/z = 297 [$M^+$], 206 [$M^+$ -$CH_2Ph$], 91 [$^+CH_2Ph$].

IR (Film): ν ($cm^{-1}$) = 3024 (C-H), 2922 (C-H), 1598 (C=C), 1046 (C-O), 696 (C-H).

$^1H$-NMR ($CDCl_3$):

δ (ppm) = 1.84 (td, J = 13.4/4.6 Hz, 2 H, $N(CH_2C\mathit{H}_2)_2$), 2.16 (dd, J = 14.3/2.1 Hz, 2 H, $N(CH_2C\mathit{H}_2)_2$), 2.37 (td, J = 12.5/2.4 Hz, 2 H, $N(C\mathit{H}_2CH_2)_2$), 2.68 (m, 2 H, $N(C\mathit{H}_2CH_2)_2$), 3.50 (s, 2 H, N-$C\mathit{H}_2$-Ph), 5.71 (d, J = 5.9 Hz, 1 H, Th-CH=CH), 6.38 (d, J = 5.9 Hz, 1 H, Th-CH=CH), 6.71 (d, J = 5.1 Hz, 1 H, 4-H-Th), 6.97 (d, J = 5.1 Hz, 1 H, 5-H-Th),

7.19 - 7.31 (m, 5 H, Ph-H).

**Example 17:** Colourless crystalline solid, Mp. 123 ˚C, yield 178 mg (55 %).

$C_{19}H_{20}N_2OS$ (324.4)

HPLC:

Method 1: 98.7 %, $R_t$ = 18.4 min
Method 2:

MS (EI): m/z = 324 [$M^+$], 298 [$M^+$ -CN], 233 [$M^+$ -$CH_2$PH], 91 [$^+CH_2$Ph].

IR (Film): ν ($cm^{-1}$) = 3090 (C-H), 2824 (C-H), 2243 (C≡N), 1067 (C-O), 696 (C-H).

$^1$H-NMR ($CDCl_3$):

δ (ppm) = 1.77 (dd, J = 13.8/2.7 Hz, 1 H, N($CH_2$C$H_2$)$_2$), 1.80-1.89 (m, 2 H, N($CH_2$C$H_2$)$_2$), 2.03 (td, J = 13.2/4.5 Hz, 1 H, N($CH_2$C$H_2$)$_2$), 2.33 (td, J = 11.8/3.1 Hz, 1 H, N(C$H_2$$CH_2$)$_2$), 2.43 (td, J = 12.6/2.7 Hz, 1 H, N(C$H_2$$CH_2$)$_2$), 2.64 - 2.75 (m, 2 H, N(C$H_2$$CH_2$)$_2$), 3.04 (dd, J = 15.6/3.9 Hz, 1 H, Th-C$H_2$CH), 3.16 (dd, J = 15.6/9.0 Hz, 1 H, Th-C$H_2$CH), 3.48 (d, J = 13.0 Hz, 1 H, N-C$H_2$-Ph), 3.53 (d, J = 13.0 Hz, 1 H, N-C$H_2$-Ph), 4.65 (dd, J = 9.0/3.3 Hz, 1 H, Th-$CH_2$C*H*), 6.75 (d, J = 5.1 Hz, 1 H, 4-H-Th), 7.09 (d, J = 5.1 Hz, 1 H, 5-H-Th), 7.19 - 7.31 (m, 5 H, Ph-*H*).

$^{13}$C-NMR ($CDCl_3$):

δ (ppm) = 30.2 (1 C, N($CH_2CH_2$)$_2$), 35.6 (1 C, N($CH_2CH_2$)$_2$), 37.8 (1 C, Th-$CH_2$CH), 49.0 (2 C, N($CH_2CH_2$)$_2$), 58.6 (1 C, Th-$CH_2$*C*H), 63.6 (1 C, N-$CH_2$-Ph), 76.4 (1 C, Th-C-O), 118.6 (1 C, CN), 124.2 (1 C, Th-CH), 124.4 (1 C, Th-CH), 127.3 (1 C, Ph-CH), 128.5 (2 C, Ph-CH), 128.8 (1 C, Ph-C), 129.5 (2 C, Ph-CH), 138.7 (1 C, Th-C), 140.5 (1 C, Th-C).

**Example 20: 1-Benzyl-6',7'-dihydrospirorpiperldin-4,4'-thieno[3,2-c]pyran]-6'-ol**

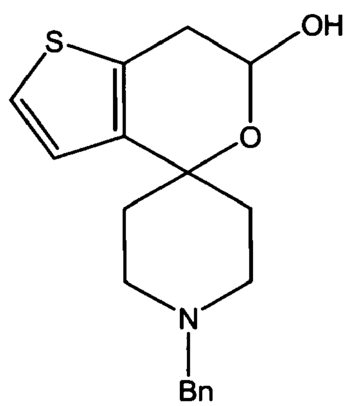

Experimental procedure

Example 3 (330 mg, 1.0 mmol) was dissolved in $CH_3CN$ (7 mL), mixed with 2M HCl (3 mL) and heated for 1 h under reflux. Following that it was alkalised with 2M NaOH (pH > 9) and extracted with $Et_2O$. For a better separation of the phases a few ml of a saturated solution of NaCl was added. The organic phases were pooled and dried over $Na_2SO_4$. Then it was filtered and the solvent removed under vacuum. The purification of the crude product was achieved by re-crystallization from $Et_2O$.

Colourless Solid, Mp. 184˚C, yield: 163g (68 %).

$C_{18}H_{21}NO_2S$ (315.4)

MS (EI): m/z =

IR (Film): ν ($cm^{-1}$) = 3100 (C-H), 1038 (C-O), 693 (C-H).

$^1$H-NMR ($CDCl_3$):

δ (ppm) = 1.76 (dd, J = 13.6/2.3 Hz, 1 H, $N(CH_2CH_2)_2$), 1.80 - 1.89 (m, 2 H, $N(CH_2CH_2)_2$), 2.04 (td, J = 13.2/4.4 Hz, 1 H, $N(CH_2CH_2)_2$), 2.37 (td, J = 11.7/4.5 Hz, 1 H, $N(CH_2CH_2)_2$), 2.43 (td, J = 11.7/2.5 Hz, 1 H, $N(CH_2CH_2)_2$), 2.62 - 2.72 (m, 2 H, $N(CH_2CH_2)_2$), 2.74 (dd, J = 15.5/7.3 Hz, Th-$CH_2$CH), 2.91 (s, 1 H, OH), 3.00 (dd, J = 15.5/3.1 Hz, 1 H, Th-$CH_2$CH), 3.48 (d, J = 14.2 Hz, 1 H, N-$CH_2$Ph), 3.51 (d, J = 14.2 Hz, 1 H, N-$CH_2$Ph), 5.25 (dd, J = 7.3/3.1 Hz, 1 H, Th-$CH_2$*CH*), 6.74 (d, J = 5.2 Hz, 1 H, 4-H-Th), 7.04 (d, J = 5.2 Hz, 1 H, 5-H-Th), 7.16 - 7.29 (m, 5 H, Ph-H).

**{1-Benzyl-6',7'-dihyrospiro[piperidin-4,4'-thieno[3,2-c]pyran]-6'-yl}acetonitril**

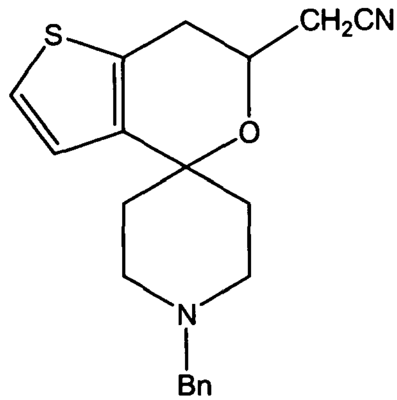

**13**

Experimental procedure

Example 20 (91 mg, 0.29 mmol) was dissolved in THF (5 mL), mixed with cyanomethylentriphenyl-phosphorane (126 mg, 0.42 mmol) und $Cs_2CO_3$ (100 mg, 0.3 mmol) and heated for 3.5 h under reflux. Subsequently $H_2O$ (10 mL) was added and a few times extracted with $CH_2Cl_2$. The pooled organic phases were dried over $Na_2SO_4$ and then filtered. After removal of the solvent under vacuum the crude product was purified using flash-chromatography (1.5 cm, cyclohexane:ethylacetate 7:3, 10 mL, $R_f$ = 0.23).

Colourless crystalline solid, Mp. 133 ˚C, yield 92 mg (94 %).

$C_{20}H_{22}N_2OS$ (338.5)

HPLC:

Method 1: 98.4 %, $R_t$ = 18.3 min

Method 2:

MS (EI): m/z = 338 [$M^+$], 298 [$M^+$ -$CH_2$CN], 247 [$M^+$ -$CH_2$Ph].

IR (Film): ν ($cm^{-1}$) = 3029 (C-H), 2923 (C-H), 2251 (C=N), 1061 (C-O), 697 (C-H).

$^1$H-NMR ($CDCl_3$):

δ (ppm) = 1.62 (dd, J = 13.5/2.6 Hz, 1 H, $N(CH_2CH_2)_2$), 1.79 (td, J = 12.6/4.4 Hz, 1 H, $N(CH_2CH_2)_2$), 1.91 (dd, J = 14.3/2.7 Hz, 1 H, $N(CH_2CH_2)_2$), 2.06 (td, J = 13.1/4.6 Hz, 1 H, $N(CH_2CH_2)_2$), 2.38 - 2.46 (m, 2 H, $N(CH_2CH_2)_2$), 2.62 - 2.70 (m, 5H (Th-$CH_2$CH$CH_2$CN) ($N(CH_2CH_2)_2$)), 2.80 (dd, J = 15.6/3.1 Hz, 1 H, Th-$CH_2$CH$CH_2$CN), 3.50 (s, 2 H, N-$CH_2$Ph), 3.98 - 4.06 (m, 1 H, Th-$CH_2$*CH*$CH_2$CN), 6.74 (d, J = 5.1 Hz, 1 H, 4-*H*-Th), 7.04 (d, J = 5.1 Hz, 1 H, 5-H-Th), 7.18 - 7.32 (m, 5 H, Ph-*H*).

**Example 19: 1-Benzyl-6',7'-dihydrospiro[piperidin-4,4'-thieno[3,2-c]pyran]**

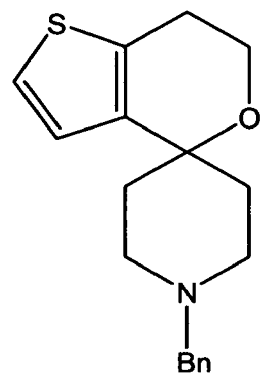

**14**

Experimental procedure

Unsaturated example 16 (60 mg, 0.2 mmol) was dissolved in a special pressure resistant flask in MeOH (8 mL) and mixed with Pd/C (15 mg, 20 % (m/m)). The solution was shaken for 24 h under a pressure of 4.5 bar $H_2$ in an hydr Hydrogenation apparatus. After the end of the reaction Pd/C was filtered off and the solvent removed under vacuum. The crude product was purified using flash-chromatography (0.7 cm, cyclohexane:ethylacetate 9:1, 5 mL, $R_f$ = 0.20).

Colourless oil, yield 53.8 mg (90 %).

$C_{18}H_{21}NOS$ (299.4)

HPLC:

Method 1: 97.2 %, $R_t$ = 18.3 min
Method 2:

MS (EI): m/z = 299 [$M^+$], 208 [$M^+$ -$CH_2$PH], 91 [$^+CH_2Ph$].

IR (Film): ν ($cm^{-1}$) = 3026 (C-H), 2922 (C-H), 1073 (C-O), 697 (C-H).

$^1$H-NMR ($CDCl_3$):

δ (ppm) = 1.77 (dd, J = 14.4/2.3 Hz, 2 H, N($CH_2CH_2$)$_2$), 1.91 (td, J = 13.4/4.5 Hz, 2 H, N($CH_2C$*$H_2$*)$_2$), 2.33 (td, J = 12.6/2.5 Hz, 2 H, N(C*$H_2$*$CH_2$)$_2$), 2.64 - 2.67 (m, 2 H, N(C*$H_2$*$CH_2$)$_2$), 2.75 (t, J = 5.4 Hz, 2 H, Th-C*$H_2$*$CH_2$), 3.49 (s, 2 H, N-C*$H_2$*Ph), 3.85 (t, J = 5.4 Hz, 2 H, Th-$CH_2$C*$H_2$*), 6.74 (d, J = 5.5 Hz, 1 H, 4-*H*-Th), 6.98 (d, J = 5.5 Hz, 1 H, 5-*H*-Th), 7.19 - 7.30 (m, 5 H, Ph-*H*).

**BIOLOGICAL ACTIVITY**

**A) In-Vitro**

Some representative compounds of the invention were tested for their activity as sigma (sigma-1 and sigma-2) inhibitors. The following protocols were followed:

**Sigma-1 (Version A)**

Brain membrane preparation and binding assays for the σ1-receptor were performed as described (DeHaven-Hudkins et al., 1992) with some modifications. In brief, guinea pig brains were homogenized in 10 vols. (w/v) of Tris-HCl 50 mM 0.32 M sucrose, pH 7.4, with a Kinematica Polytron PT 3000 at 15000 r.p.m. for 30 s. The homogenate was centrifuged at 1000g for 10 min at 4°C and the supernatants collected and centrifuged again at 48000g for 15 min at 4°C. The pellet was resuspended in 10 volumes of Tris-HCl buffer (50 mM, pH 7.4), incubated at 37°C for 30 min, and

centrifuged at 48000g for 20 min at 4°C. Following this, the pellet was resuspended in fresh Tris-HCl buffer (50 mM, pH 7.4) and stored on ice until use.

Each assay tube contained 10 μL of [$^3$H](+)-pentazocine (final concentration of 0.5 nM), 900 μL of the tissue suspension to a final assay volume of 1 mL and a final tissue concentration of approximately 30 mg tissue net weight/mL. Non-specific binding was defined by addition of a final concentration of 1 μM haloperidol. All tubes were incubated at 37°C for 150 min before termination of the reaction by rapid filtration over Schleicher & Schuell GF 3362 glass fibre filters [previously soaked in a solution of 0,5% polyethylenimine for at least 1 h]. Filters were then washed with four times with 4 mL of cold Tris-HCl buffer (50 mM, pH 7.4). Following addition of scintillation cocktail, the samples were allowed to equilibrate overnight. The amount of bound radioactivity was determined by liquid scintillation spectrometry using a Wallac Winspectral 1414 liquid scintillation counter. Protein concentrations were determined by the method of Lowry et al. (1951).

**Sigma 1 (Version B)**

In brief the $\sigma_1$-receptor preparation was prepared from guinea pig brain. The brains were homogenized in 5 to 6 times of volume sucrose solution (0.32M) and homogenized. The homogenate was centrifuged at 2900 rpm, 4°C, 10 min). the supernatant was centrifuged again (23500 x g, 4°C, 20 min). The pellet was resuspended in Tris-buffer, incubated for 30 min at room temperature and centrifuged f (23500 x g, 4°C, 20 min). The pellet was resuspended in cold TRIS-buffer and homogenized. Then the protein content was measured (approx. 1.5 mg/mL) and the homogenate frozen at -80°C for later use.

The radioligand used was [$^3$H]-(+)-Pentazocin in TRIS-buffer. In a volume of 200 μL 50 μL TRIS-buffer, 50 μL compound solution of varying concentration, 50 μL radioligand- solution (8 nM; resulting in 2 nM in the assay) and finally 50 μL of receptor preparation (approx. 1.5 mg /mL) were given into a well of a microplate equipped with a filter. The plate was closed and stirred for 2.5 h at 37 °C and 500 rpm. Following that the solvents were removed by a harvester through the filter. After rinsing with $H_2O$ the filter was measured in a scintillation counter ([$^3$H]-protocol).

**Sigma-2 (Version A)**

Binding studies for σ2-receptor were performed as described (Radesca et al., 1991) with some modifications. In brief, brains from sigma receptor type I (σ1) knockout mice were homogenized in a volume of 10 mL/g tissue net weight of ice-cold 10 mM Tris-HCl, pH 7.4, containing 320 mM sucrose (Tris-sucrose buffer) with a Potter-Elvehjem homogenizer (10 strokes at 500 r.p.m.) The homogenates were then centrifuged at 1000g for 10 min at 4°C, and the supernatants were saved. The pellets were resuspended by vortexing in 2 mUg ice-cold Tris-sucrose buffer and centrifuged again at 1000g for 10 min. The combined 1000g supernatants were centrifuged at 31000g for 15 min at 4°C. The pellets were resuspended by vortexing in 3 mUg 10 mM Tris-HCl, pH 7.4, and the suspension was kept at 25°C for 15 min. Following centrifugation at 31000g for 15 min, the pellets were resuspended by gentle Potter Elvehjem homogenization to a volume of 1.53 mUg in 10 mM Tris-HCl pH 7.4.

The assay tubes contained 10 μL of [$^3$H]-DTG (final concentration of 3 nM), 400 μL of the tissue suspension (5.3 mUg in 50 mM Tris-HCl, pH 8.0) to a final assay volume of 0.5 mL. Non-specific binding was defined by addition of a final concentration of 1 μM haloperidol. All tubes were incubated at 25°C for 120 min before termination of the reaction by rapid filtration over Schleicher & Schuell GF 3362 glass fibre filters [previously soaked in a solution of 0,5% polyethylenimine for at least 1 h]. Filters were washed with three times with 5 mL volumes of cold Tris-HCl buffer (10 mM, pH 8.0). Following addition of scintillation cocktail samples were allowed to equilibrate overnight. The amount of bound radioactivity was determined by liquid scintillation spectrometry using a Wallac Winspectral 1414 liquid scintillation counter. Protein concentrations were determined by the method of Lowry et al. (1951).

**References**

DeHaven-Hudkins, D. L., L.C. Fleissner, and F. Y. Ford-Rice, 1992, "Characterization of the binding of [3H](+) pentazocine to σ recognition sites in guinea pig brain", Eur. J. Pharmacol. 227, 371-378.

Radesca, L., W.D. Bowen, and L. Di Paolo, B.R. de Costa, 1991, Synthesis and Receptor Binding of Enantiomeric N-Substituted cis-N-[2-(3,4-Dichlorophenyl)ethyl]-2-(1-pyrrolidinyl)cyclohexylamines as High-Affinity σ Receptor Ligands, J. Med. Chem. 34, 3065-3074.

Langa, F., Codony X., Tovar V., Lavado A., Giménez E., Cozar P., Cantero M., Dordal A., Hernández E., Pérez R., Monroy X., Zamanillo D., Guitart X., Montoliu Ll., 2003, Generation and phenotypic analysis of sigma receptor type I (Sigma1) knockout mice, European Journal of Neuroscience, Vol. 18, 2188-2196.

Lowry, O.H., N.J. Rosebrough, A.L. Farr, and R.J. Randall, 1951, Protein measurement with the Folin phenol reagent, J. Biol. Chem, 193, 265.

## SIGMA 2 (Version B)

In brief the $\sigma_2$-Receptor preparation was prepared from rat liver. The livers were homogenized in 5 to 6 times of volume sucrose solution (0.32M) and homogenized. The homogenate was centrifuged at 2900 rpm, 4°C, 10 min). the supernatant was centrifuged again (31000 x g, 4°C, 20 min). The pellet was resuspended in TRIS-buffer, incubated for 30 min at room temperature while stirring and centrifuged f (31000 x g, 4°C, 20 min). The pellet was resuspended in cold TRIS-buffer pH 8 and homogenized. Then the protein content was measured (approx. 2 mg/mL) and the homogenate frozen at -80°C for later use.

The radioligand used was [$^3$H]-Ditolylguanidin in TRIS-buffer pH 8. The $\sigma_1$-receptor binding sites were masked through (+)-Pentazocin-solution in TRIS-Puffer pH 8.

In a volume of 200 µL 50 µL compound solution of varying concentration, 50 µL (+)-Pentazocin- solution (2 µM; resulting in 500 nM in the assay), 50 µL radioligand-solution (12 nM; resulting in 3 nM in the assay) and finally 50 µL of receptor preparation (approx. 2 mg /mL) were given into a well of a microplate equipped with a filter. The plate was closed and stirred for 2 h at room temperature and 500 rpm. Following that the solvents were removed by a harvester through the filter. After rinsing with $H_2O$ the filter was measured in a scintillation counter ([$^3$H]-protocol).

Some of the results obtained (through the version B) are shown in table (I).

**Table (I)**

| Example | Formula | Binding σ1 Ki [nM] | Binding σ2 Ki [nM] or *[Inhibition at 1µM] |
|---|---|---|---|
| 2 | 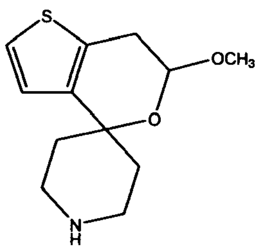 | 602 | 17%* |
| 3 | 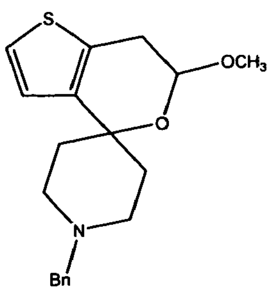 | 0.32±0.16 | 1260 |
| 4 | 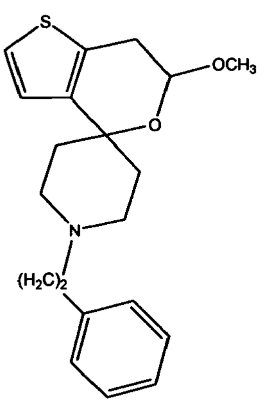 | 5.89 ± 5.10 | 191 |

(continued)

| Example | Formula | Binding σ1 Ki [nM] | Binding σ2 Ki [nM] or *[Inhibition at 1μM] |
|---|---|---|---|
| 5 | 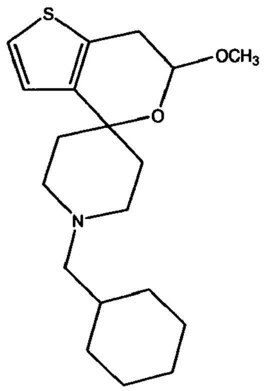 | 0.29 ± 0.079 | 25.4 ± 13.5 |
| 6 | 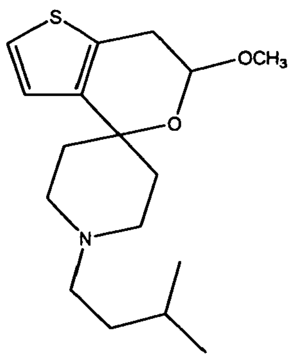 | 0.96 ± 0.53 | 285 |
| 7 | 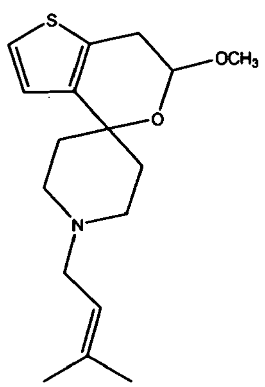 | 0.96±0.25 | 78.1±74.0 |
| 8 | 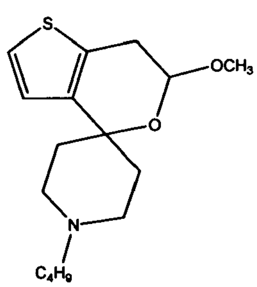 | 4.30 ± 0.44 | 497 ± 294 |

(continued)

| Example | Formula | Binding σ1 Ki [nM] | Binding σ2 Ki [nM] or *[Inhibition at 1μM] |
|---|---|---|---|
| 9 | 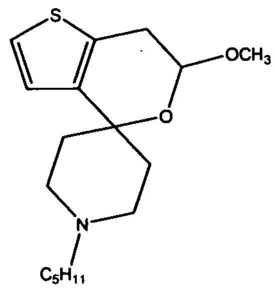 | 1.64 ± 0.53 | 460 |
| 10 | 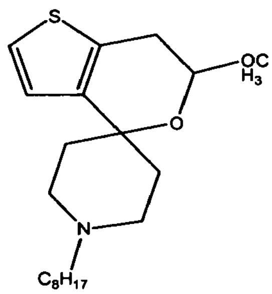 | 4.5 ± 2.0 | 1200 |
| 11 | 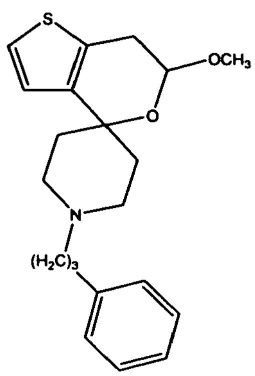 | 2.42±1.66 | 203 |
| 12 | 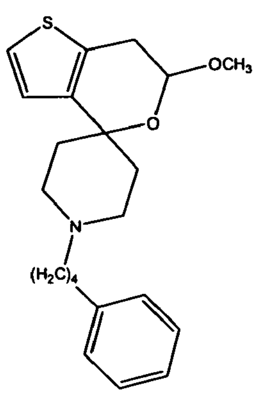 | 7.67±2.15 | 99.2±21.9 |

(continued)

| Example | Formula | Binding σ1 Ki [nM] | Binding σ2 Ki [nM] or *[Inhibition at 1μM] |
|---|---|---|---|
| 13 | 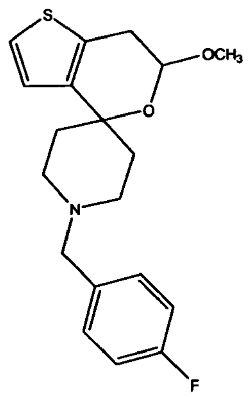 | 3.39±4.54 | 425 |
| 14 | 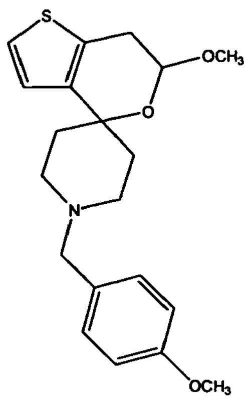 | 2.24 ± 0.99 | 2970 |
| 15 | 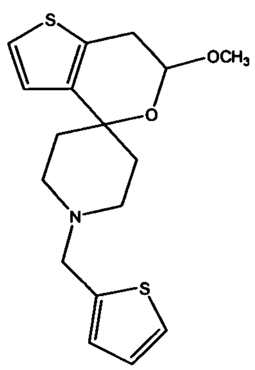 | 2.02 ± 0.61 | 1040 |
| 16 | 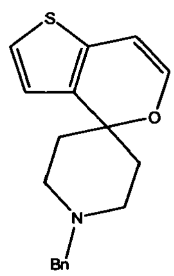 | 1.98 ± 0.89 | 41.9 ± 43.4 |

(continued)

| Example | Formula | Binding σ1 Ki [nM] | Binding σ2 Ki [nM] or *[Inhibition at 1μM] |
|---|---|---|---|
| 17 | 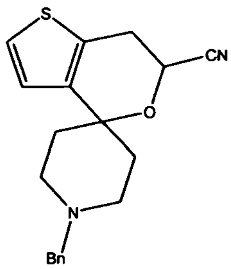 | 1.40 ± 0.32 | 783 |
| 18 | 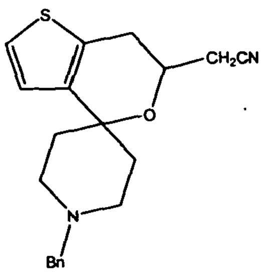 | 3.14±3.33 | 6%* |
| 19 | 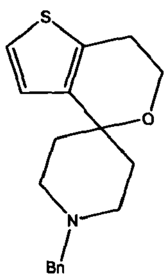 | 0.31 ± 0.11 | 9.6 ± 8.7 |

**B) In-Vivo**

**EFFECT ON CAPSAICIN IN DEVELOPMENT OF MECHANICAL ALLODYNIA**

This model uses the von-Frey Filaments and is a model to test the effects or symptoms of neuropathic pain, allodynia etc.

Interest of the model:

- The injection of 1 μg of capsaicin to experimental animals produces acute pain followed by hyperalgesia/allodynia
- The mechanisms involved in capsaicin-induced acute pain and hyperalgesia are relatively well known (mainly activation of peripheral nociceptors and sensitization of spinal cord neurons, respectively)

The test protocol for all tests with of Frey filaments: After habituation mice were first treated with the test-compound (or solvent in controls). Then 1 μg capsaicin (1% DMSO) is injected into their paw resulting in developing pain in the effected paw. The effected paw is then treated with a mechanical stimulus and the latency time before the paw is withdrawn is measured.

Using this pharmacological test the compounds according to example 22 and example 25 showed a clear antiallodynic effect. They were tested at 16 mg/kg i.p. in 8 animals each and respectively achieved 45.67 ± 9.62 % and 44.28 ± 9.49 % of pain relief.

## Claims

**1.** Compound of general formula (I) or (Ia),

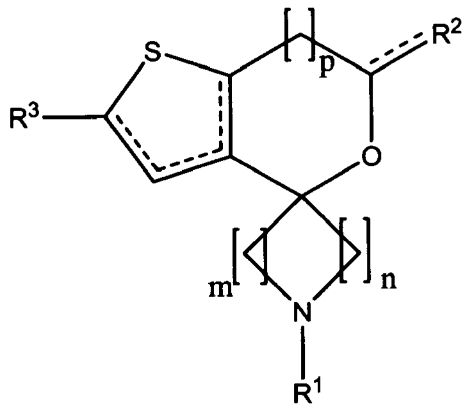

or

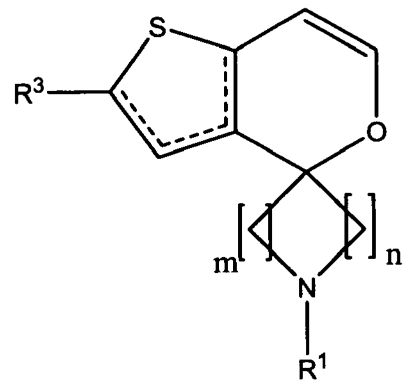

(I) (Ia)

wherein

m is selected from 1, 2 or 3, n is selected from 1, 2 or 3, and m + n is either 3, 4 or 5;
p is selected from 0 or 1;

the dotted lines are either a double or a single bond;
$R^1$ is selected from hydrogen; an optionally at least monosubstituted, linear or branched $C_{1-18}$-aliphatic group; an optionally at least monosubstituted aryl; an optionally at least monosubstituted heterocyclyl; an optionally at least monosubstituted cycloalkyl; an optionally at least monosubstituted alkyl-aryl; an optionally at least monosubstituted alkyl-heterocyclyl; or an optionally at least monosubstituted alkyl-cycloalkyl;
$R^2$ is selected from hydrogen; an optionally at least monosubstituted, linear or branched $C_{1-6}$-aliphatic group; $(CH_2)_qCN$ with q being 0, 1, 2, 3 or 4; O-R with R being H or an optionally at least monosubstituted, linear or branched $C_{1-6}$-aliphatic group; =O; $(CH_2)_sCOR^*$; $(CH_2)_sNR^*R^{**}$; $CONR^*R^{**}$; $(CH_2)_sCO_2R^*$; $CH{=}NOR^*$; $(CH_2)_sOR^*$;
with

R* being H; or an optionally at least monosubstituted, linear or branched $C_{1-6}$-aliphatic group;
R** being an optionally at least monosubstituted, linear or branched $C_{1-6}$-aliphatic group;
s being 0, 1, or 2;

$R^3$ is selected from hydrogen; an optionally at least monosubstituted, linear or branched $C_{1-18}$-aliphatic group; $NO_2$; $SO_3H$; COR'''; $(CH_2)_rNR'R''$; $CO_2R'$; an optionally at least monosubstituted alkyl-aryl with alkyl optionally being substituted by OH; an optionally at least monosubstituted alkyl-heterocyclyl with alkyl optionally being substituted by OH; or an optionally at least monosubstituted alkyl-cycloalkyl with alkyl optionally being substituted by OH;
with

R' being H; or an optionally at least monosubstituted, linear or branched $C_{1-6}$-aliphatic group;
R'' being an optionally at least monosubstituted, linear or branched $C_{1-6}$-aliphatic group;
R''' being H; an optionally at least monosubstituted, linear or branched $C_{1-6}$-aliphatic group; or an optionally at least monosubstituted aryl, an optionally at least monosubstituted heterocyclyl, or an optionally at least monosubstituted cycloalkyl;
r being 0, 1, or 2;

optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

**2.** Compound according to claim 1, **characterized in that** $R^1$ is selected from H; an optionally at least monosubstituted, linear or branched $C_{1-8}$-aliphatic group; an optionally at least monosubstituted alkyl-aryl; an optionally at least monosubstituted alkyl-heterocyclyl; or an optionally at least monosubstituted alkyl-cycloalkyl.

**3.** Compound according to any of claims 1 or 2, **characterized in that** $R^2$ is selected from H; $(CH_2)_qCN$ with q being 0, 1, 2, 3 or 4; or OR with R being H or an optionally at least monosubstituted, linear or branched $C_{1-6}$-aliphatic group; preferably $R^2$ is selected from H; CN; $CH_2CN$; or OR with R being H or a linear or branched $C_{1-4}$-alkyl group, more preferably $R^2$ is selected from H, CN; $CH_2CN$; OH or $OCH_3$.

**4.** Compound according to any of claims 1 to 3, **characterized in that** $R^3$ is selected from H; or an optionally at least monosubstituted, linear or branched $C_{1-6}$-aliphatic group; especially $R^3$ is selected from H; or an optionally at least monosubstituted, linear or branched $C_{1-6}$-alkyl group.

**5.** Compound according to any of claims 1 to 4, **characterized in that** m and n are selected from 1 or 2 and m+n are selected from 3 or 4.

**6.** Compound according to claim 1, having a general formula **Ib,**

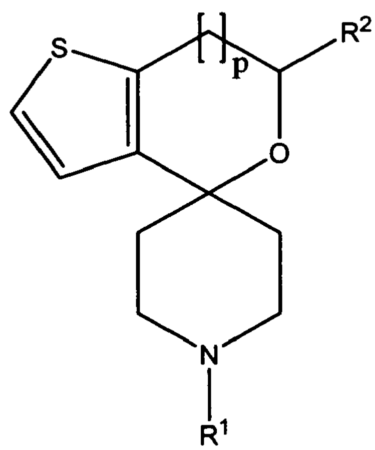

**(Ib)**

wherein

p is selected from 0 or 1;
$R^1$ is selected from hydrogen; an optionally at least monosubstituted, linear or branched $C_{1-18}$-aliphatic group; an optionally at least monosubstituted aryl; an optionally at least monosubstituted heterocyclyl; an optionally at least monosubstituted cycloalkyl; an optionally at least monosubstituted alkyl-aryl; an optionally at least monosubstituted alkyl-heterocyclyl; an optionally at least monosubstituted alkyl-cycloalkyl; or COOR' with R' being either H or $C_{1-4}$-alkyl;
$R^2$ is selected from hydrogen; an optionally at least monosubstituted, linear or branched $C_{1-6}$-aliphatic group; $(CH_2)_qCN$ with q being 0, or 1; or OR with R being H or an optionally at least monosubstituted, linear or branched $C_{1-6}$-aliphatic group;

optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

**7.** Compound according to claim 6, **characterized in that** $R^1$ is selected from hydrogen; an optionally at least monosubstituted, linear or branched $C_{1-18}$-aliphatic group; an optionally at least monosubstituted alkyl-aryl; an optionally at least monosubstituted alkyl-heterocyclyl; or an optionally at least monosubstituted alkyl-cycloalkyl.

**8.** Compound according to any of claims 6 or 7, **characterized in that** $R^2$ is selected from H; $CH_2CN$; CN; or OR with R being H or an optionally at least monosubstituted, linear or branched $C_{1-6}$-alkyl group; especially $R^2$ is selected from H; $CH_2CN$; CN; OH or a linear or branched $OC_{1-4}$-alkyl group; more especially $R^2$ is selected from H, CN, $CH_2CN$, OH or $OCH_3$.

**9.** Compound according to any of claims 6 to 8, **characterized in that** $R^1$ is selected from

hydrogen, $C_{1-10}$-alkyl, linear or branched; $C_{1-10}$-alkenyl, linear or branched; optionally at least mono-substituted $C_{1-6}$-alkyl-aryl; optionally at least mono-substituted $C_{1-6}$-alkyl-heterocyclyl; or optionally at least mono-substituted $C_{1-6}$-alkyl-cycloalkyl;
preferably $R^1$ is selected from hydrogen; $C_{1-10}$-alkyl, linear or branched; $C_{2-8}$-alkenyl, linear or branched; optionally at least mono-substituted $C_{1-6}$-alkyl-aryl; optionally at least mono-substituted $C_{1-6}$-alkyl-heterocyclyl; or optionally at least mono-substituted $C_{1-6}$-alkyl-$C_{4-8}$-cycloalkyl.

**10.** Compound according to claim 1 or 6, having a general formula Ib,

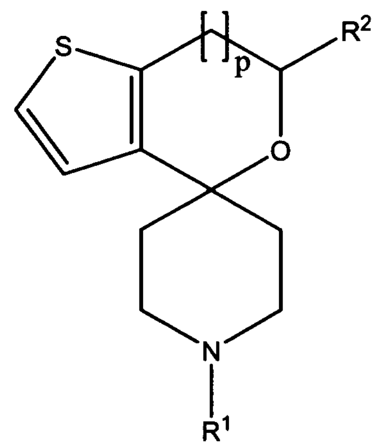

**(Ib)**

wherein

p is selected from 0 or 1;
$R^1$ is selected from hydrogen; $C_{1-10}$-alkyl, linear or branched; $C_{2-8}$-alkenyl, linear or branched; optionally at least mono-substituted $C_{1-6}$-alkyl-aryl; optionally at least mono-substituted $C_{1-6}$-alkyl-heterocyclyl; or optionally at least mono-substituted $C_{1-6}$-alkyl-$C_{4-8}$-cycloalkyl;
$R^2$ is selected from H; CN; $CH_2CN$; OH or a linear or branched $OC_{1-4}$-alkyl group;

optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

**11.** Compound according to claim 1, **characterized in that** the compound is selected from

- Ethyl-6'-methoxy-6',7'-dihydrospiro[piperidin-4,4'-thieno[3,2-c]pyran]-1-carboxylate;
- 6'-Methoxy-6',7'-dihydrospiro[piperidin-4,4'-thieno[3,2-c]pyran;
- 1-Benzyl-6'-methoxy-6',7'-dihydrospiro[piperidin-4,4'-thieno[3.2-c]pyran];
- 6'-Methoxy-1-(2-phenylethyl)-6',7'-dihydrospiro[piperidin-4,4'-thieno[3.2-c]pyran];
- 1-(Cyclohexylmethyl)-6'-Methoxy -6',7'-dihydrospiro[piperidin-4,4'-thieno[3.2-c]pyran];
- 6'-Methoxy-1-(3-methylbutyl)-6',7'-dihydrospiro[piperidin-4,4'-thieno[3.2-c]pyran];
- 6'-Methoxy-1-(3-methylbut-2-enyl)-6',7'-dihydrospiro[piperidin-4,4'-thieno[3.2-c]pyran];
- 1-Butyl-6'-methoxy-6',7'-dihydrospiro[piperidin-4,4'-thieno[3.2-c]pyran];
- 6'-Methoxy-1-pentyl-6',7'-dihydrospiro[piperidin-4,4'-thieno[3.2-c]pyran];
- 6'-Methoxy-1-octyl -6',7'-dihydrospiro[piperidin-4,4'-thieno[3.2-c]pyran];
- 3'-Methoxy-1-(3-phenylpropyl)-6',7'-dihydrospiro[piperidin-4,4'-thieno[3.2-c]pyran];
- 3'-Methoxy-1-(4-phenylbutyl)-6',7'-dihydrospiro[piperidin-4,4'-thieno[3.2-c]pyran];
- 1-(4-Fluorbenzyl)-6'-Methoxy -6',7'-dihydrospiro[piperidin-4,4'-thieno[3.2-c]pyran];
- 6'-Methoxy-1-(4-methoxybenzyl)-6',7'-dihydrospiro[piperidin-4,4'-thieno[3.2-c]pyran];
- 6'-Methoxy-1-(thien-2-ylmethyl)-6',7'-dihydrospiro[piperidin-4,4'-thieno[3.2-c]pyran];
- 1-Benzylspiro[piperidin-4,4'-thieno[3,2-c]pyran];
- 1-Benzyl-6',7'-dihydrospiro[piperidin-4,4'-thieno[3,2-c]pyran]-6'-carbonitrile;
- {1-Benzyl-6',7'-dihyrospiro[piperidin-4,4'-thieno[3,2-c]pyran]-6'-yl}acetonitrile;
- 1-Benzy)-6',7'-dihydrospiro[piperidin-4,4'-thieno[3,2-c]pyran]; or
- 1-Benzyl-6',7'-dihydrospiro[piperidin-4,4'-thieno[3,2-c]pyran]-6'-ol;

optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof;
preferably from

- 1-Benzyl-6'-methoxy-6',7'-dihydrospiro[piperidin-4,4'-thieno[3.2-c]pyran];
- 6'-Methoxy-1-(2-phenylethyl)-6',7'-dihydrospiro[piperidin-4,4'-thieno[3.2-c]pyran];
- 1-(Cyclohexylmethyl)-6'-Methoxy -6',7'-dihydrospiro[piperidin-4,4'-thieno[3.2-c]pyran];
- 6'-Methoxy-1-(3-methylbutyl)-6',7'-dihydrospiro[piperidin-4,4'-thieno[3.2-c]pyran];
- 6'-Methoxy-1-(3-methylbut-2-enyl)-6',7'-dihydrospiro[piperidin-4,4'-thieno[3.2-c]pyran];
- 1-Butyl-6'-methoxy-6',7'-dihydrospiro[piperidin-4,4'-thieno[3.2-c]pyran];
- 6'-Methoxy-1-pentyl-6',7'-dihydrospiro[piperidin-4,4'-thieno[3.2-c]pyran];
- 6'-Methoxy-1-octyl -6',7'-dihydrospiro[piperidin-4,4'-thieno[3.2-c]pyran];
- 3'-Methoxy-1-(3-phenylpropyl)-6',7'-dihydrospiro[piperidin-4,4'-thieno[3.2-c]pyran];
- 3'-Methoxy-1-(4-phenylbutyl)-6',7'-dihydrospiro[piperidin-4,4'-thieno[3.2-c]pyran];
- 1-(4-F)uorbenzy))-6'-Methoxy-6',7'-dihydrospiro[piperidin-4,4'-thieno[3.2-c]pyran];
- 6'-Methoxy-1-(4-methoxybenzyl)-6',7'-dihydrospiro[piperidin-4,4'-thieno[3.2-c]pyran];
- 6'-Methoxy-1-(thien-2-ylmethyl)-6',7'-dihydrospiro[piperidin-4,4'-thieno[3.2-c]pyran];
- 1-Benzyl-6',7'-dihydrospiro[piperidin-4,4'-thieno[3,2-c]pyran]-6'-carbonitrile;
- {1-Benzyl-6',7'-dihyrospiro[piperidin-4,4'-thieno[3,2-c]pyran]-6'-yl}acetonitrile;
- 1-Benzyl-6',7'-dihydrospiro[piperidin-4,4'-thieno[3,2-c]pyran]; or
- 1-Benzyl-6',7'-dihydrospiro[piperidin-4,4'-thieno[3,2-c]pyran]-6'-ol;

optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

**12.** Process for the production of a compound according to claim 1, wherein a compound of formula (III)

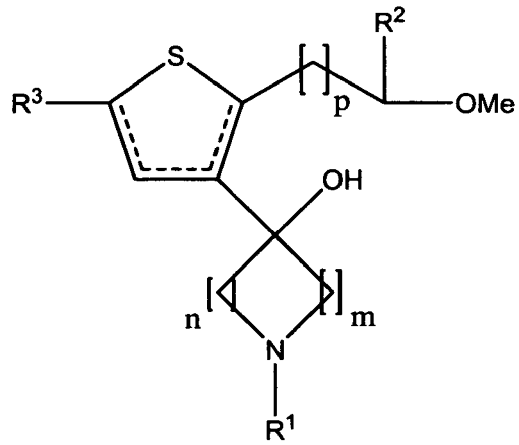

(III)

, wherein $R^1$, $R^2$, $R^3$, m, n and p are as defined in claim 1 is reacted with p-toluol sulphonic acid to form acompound according the formula (I).

**13.** Process according to claim 12, wherein

- $R^2$ is $OCH_3$; and/or
- $R^3$ is H and/or
- p is H.

**14.** Process for the production of a compound according to formula Ib, wherein a compound of formula (IIIb)

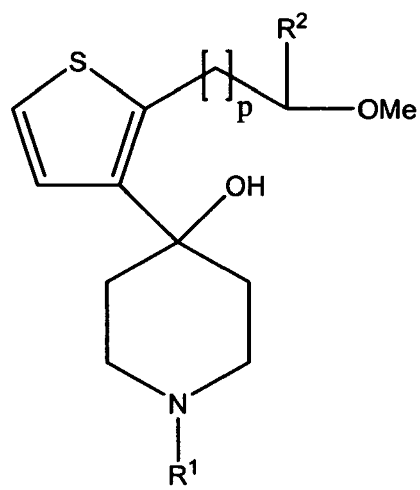

(IIIb)

, wherein $R^1$, $R^2$ and p are as defined in claim 1 is reacted with p-toluol sulphonic acid to form acompound according the formula (Ib).

**15.** Process according to claim 14, wherein

- $R^2$ is $OCH_3$ and/or
- p is 1.

**16.** Process according to claims 12 to 15, wherein as a next step a compound according to formula (I) or (Ib) in which $R^3$ is C(O)OR' is reacted with a alkaline solution in water to form a compound according to formulas (I) or (Ib) with $R^1$ being H.

**17.** A pharmaceutical composition which comprises a compound as defined in any of claims 1 to 11 or a pharmaceutically acceptable salt, prodrug, isomer or solvate thereof, and a pharmaceutically acceptable carrier, adjuvant or vehicle.

**18.** Use of a compound as defined in any of claims 1 to 11 in the manufacture of a medicament.

**19.** Use of a compound as defined in any of claims 1 to 11 in the manufacture of a medicament for the treatment or prophylaxis of a sigma receptor mediated disease or condition.

**20.** Use according to claim 19 wherein the disease is diarrhoea, lipoprotein disorders, metabolic syndrome, treatment of elevated triglyceride levels, chylomicronemia, hyperlipoproteinemia; hyperlipidemia, especially mixed hyperlipidemia; hypercholesterolemia, dysbetalipoproteinemia, hypertriglyceridemia including both the sporadic and familial disorder (inherited hypertriglyceridemia), migraine, obesity, arthritis, hypertension, arrhythmia, ulcer, learning, memory and attention deficits, cognition disorders, neurodegenerative diseases, demyelinating diseases, addiction to drugs and chemical substances including cocaine, amphetamine, ethanol and nicotine, tardive diskinesia, ischemic stroke, epilepsy, stroke, depression, stress, psychotic condition, schizophrenia; inflammation, autoimmune diseases or cancer.

**21.** Use according to claim 19 wherein the disease is pain, especially neuropathic pain, inflammatory pain or other pain conditions, allodynia and/or hyperalgesia, especially mechanical allodynia.

**22.** Use of a compound as defined in any of claims 1 to 11 as pharmacological tool or as anxiolytic or immunosuppressant.

European Patent Office

# EUROPEAN SEARCH REPORT

Application Number
EP 07 38 4027

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 97/11697 A (MERCK & CO INC [US]) 3 April 1997 (1997-04-03)<br>* page 34; compounds 1.,2. *<br>* page 57 - page 58; compounds 25,27 *<br>* page 165 - page 169; examples B4,B5,intermediates *<br>* page 76 - page 78 *<br>* claims 1,7,14 *<br>----- | 1-22 | INV.<br>C07D495/20<br>A61K31/438<br>A61P25/18 |
| X | US 4 021 451 A (AMERICAN HOME PROD CORP [US]) 3 May 1977 (1977-05-03)<br>* column 3, line 25 - line 30 *<br>* example 48 *<br>----- | 1-16,<br>18-22 | |
| X | DATABASE CA [Online]<br>CHEMICAL ABSTRACTS SERVICE, COLUMBUS, OHIO, US; 1988,<br>SAUTER, FRITZ ET AL: "Thienospirans. V. Thienospirans via directed lithiations"<br>XP002448343<br>retrieved from STN<br>Database accession no. 1988:221581<br>* abstract *<br>& HETEROCYCLES , 26(10), 2639-56 CODEN: HTCYAM; ISSN: 0385-5414, 1987,<br>-----<br>-/-- | 1-16 | TECHNICAL FIELDS SEARCHED (IPC)<br>C07D<br>A61K<br>A61P |

The present search report has been drawn up for all claims

| Place of search | Date of completion of the search | Examiner |
|---|---|---|
| Munich | 28 August 2007 | Cortés, José |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

2

EPO FORM 1503 03.82 (P04C01)

European Patent Office

# EUROPEAN SEARCH REPORT

Application Number
EP 07 38 4027

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DATABASE CA [Online] CHEMICAL ABSTRACTS SERVICE, COLUMBUS, OHIO, US; 1975, DOBSON, THOMAS A. ET AL: "Pyrano heterocycles. I. Syntheses of isochromans and the novel thieno[3,2-c]pyran, benzothieno[3,2-c]pyran, benzothieno[2,3-c]pyran, and pyrano[4,3-b]benzofuran systems" XP002448344 retrieved from STN Database accession no. 1975:514254 * abstract * & JOURNAL OF HETEROCYCLIC CHEMISTRY , 12(3), 591-4 CODEN: JHTCAD; ISSN: 0022-152X, 1975, ----- | 1-16 | |
| A | WO 01/12630 A (SEPRACOR INC [US]) 22 February 2001 (2001-02-22) * page 8 * * examples * * claims * ----- | 1-22 | TECHNICAL FIELDS SEARCHED (IPC) |

The present search report has been drawn up for all claims

| Place of search | Date of completion of the search | Examiner |
|---|---|---|
| Munich | 28 August 2007 | Cortés, José |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

2

EPO FORM 1503 03.82 (P04C01)

**ANNEX TO THE EUROPEAN SEARCH REPORT
ON EUROPEAN PATENT APPLICATION NO.** EP 07 38 4027

This annex lists the patent family members relating to the patent documents cited in the above-mentioned European search report.
The members are as contained in the European Patent Office EDP file on
The European Patent Office is in no way liable for these particulars which are merely given for the purpose of information.

28-08-2007

| Patent document cited in search report | | Publication date | Patent family member(s) | | Publication date |
|---|---|---|---|---|---|
| WO 9711697 | A | 03-04-1997 | AU | 7169696 A | 17-04-1997 |
| US 4021451 | A | 03-05-1977 | NONE | | |
| WO 0112630 | A | 22-02-2001 | AU | 6525300 A | 13-03-2001 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2003035065 A
- WO 2007001939 A
- FR 2875230


### Non-patent literature cited in the description


- **WALKER, J.M. et al.** *Pharmacological Reviews,* 1990, vol. 42, 355
- **SNYDER, S.H. ; LARGENT, B.L.** *J. Neuropsychiatry,* 1989, vol. 1, 7
- **QUIRION, R. et al.** *Trends Pharmacol. Sci.,* 1992, vol. 13, 85-86
- **HANNER, M. et al.** *Proc. Natl. Acad. Sci.,* 1996, vol. 93, 8072-8077
- **MAIER et al.** *J Med Chem,* 2002, vol. 45, 438-448
- **MAIER et al.** *J Med Chem,* 2002, vol. 45, 4923-4930
- **KROGSGAARD-LARSEN et al.** Textbook of Drug design and Discovery. Taylor & Francis, April 2002
- **DEHAVEN-HUDKINS, D. L. ; L.C. FLEISSNER ; F. Y. FORD-RICE.** Characterization of the binding of [3H](+)pentazocine to σ recognition sites in guinea pig brain. *Eur. J. Pharmacol.,* 1992, vol. 227, 371-378
- **RADESCA, L. ; W.D. BOWEN ; L. DI PAOLO ; B.R. DE COSTA.** Synthesis and Receptor Binding of EnantiomericN-Substitutedcis-N-[2-(3,4-Dichlorophenyl)ethyl]-2-(1-pyrrolidinyl)cyclohexylamines as High-Affinity σ Receptor Ligands. *J. Med. Chem.,* 1991, vol. 34, 3065-3074
- **LANGA, F. ; CODONY X. ; TOVAR V. ; LAVADO A. ; GIMÉNEZ E. ; COZAR P. ; CANTERO M. ; DORDAL A. ; HERNÁNDEZ E. ; PÉREZ R.** Generation and phenotypic analysis of sigma receptor type I (Sigma1) knockout mice. *European Journal of Neuroscience,* 2003, vol. 18, 2188-2196
- **LOWRY, O.H. ; N.J. ROSEBROUGH ; A.L. FARR ; R.J. RANDALL.** Protein measurement with the Folin phenol reagent. *J. Biol. Chem,* 1951, vol. 193, 265